# EUROPEAN PATENT APPLICATION

(11) **EP 2 311 865 A1**
(43) Date of publication of application: **20.04.2011**
(21) Application number: 10191911.6
(22) Date of filing: 19.11.2010
(51) Int. Cl.: C07K 14/47, G01N 33/574, G01N 33/68

(54) **Placental Growth Factor in Cancer Disease**

(71) Applicant: F. Hoffmann-La Roche AG, 4070 Basel (CH); Roche Diagnostics GmbH, 68305 Mannheim (DE)
(72) Inventor: The designation of the inventor has not yet been filed
(74) Representative: Huhn, Michael

(57) **Abstract**

The present invention relates to the field of laboratory diagnostics. Specifically means and methods for diagnosing a tumor disease and for monitoring anti-tumor therapy with a VEGF-inhibitor are disclosed. The invention uses placental growth factor (PIGF) or a variant thereof, a cardiac troponin or a variant thereof, optionally hepatocyte growth factor (HGF) or a variant thereof, and/or soluble fms-like tyrosine kinase-1 (sFIT-1) as biomarkers for the aforementioned purposes.

## Description

The present invention relates to the field of laboratory diagnostics. Specifically means and methods for diagnosing a tumor disease and for monitoring anti-tumor- therapy with a VEGF-inhibitor are disclosed. The invention uses placental growth factor (PlGF) or a variant thereof, hepatocyte growth factor (HGF) or a variant thereof, soluble fms-like tyrosine kinase-1 (sFlT-1) and a cardiac troponin or a variant thereof as biomarkers for the aforementioned purposes.

For a long time, treatment of cancer has relied on three approaches: surgical resection of the tumor or a part of it, radiation therapy with ionizing radiation and chemotherapy. Chemotherapy used to be the only therapy that offered hope for a cure of metastatic cancer because the effects of surgery and radiation therapy are localized. However, in most cancers chemotherapy does not achieve a cure although it may delay tumor progression and increase the life expectancy of the patient. Chemotherapy has been hampered by side effects caused by the often highly toxic medications and by the resistance of tumor cells. Recently, the concept of anti-angiogenic therapy has been put to practice. Since each tumor beyond a certain size requires vascularization for its supply with oxygen and nutrients, the inhibition of angiogenesis promises to "starve" the tumor. Currently, most methods of anti-angiogenic therapy are directed at vascular endothelial growth factor (VEGF). However, anti-VEGF-therapy is not always successful. Anti-VEGF therapy may fail to inhibit VEGF-mediated angiogenesis. Alternatively, the tumor may be able to utilize VEGF-independent pathways to promote vascularization. Moreover, as set forth above, anti-VEGF-therapy may interfere with the establishment of collateral circulation around blocked blood vessels, thus aggravating the effects of cardiac diseases.

The available methods for monitoring course of a cancer disease or the effects of anti-cancer therapy are limited. Tumor response to therapy is often determined by the disappearance of the tumor or significant size reduction. However, while this criterion may be suitable for monitoring treatment with cytotoxic drugs (which kill at least part of the tumor cells), it is not suitable for monitoring treatment with cytostatic drugs (which do not directly reduce tumor mass). Therefore, tumor shrinkage is in many cases an insensitive estimate of efficacy of anti-cancer-therapy (Jubb et al., 2006, Nature Reviews - Cancer, 6: 626-635). Tumor shrinkage can be controlled by computed tomography (CT) or ultrasound. However, CT and ultrasound only give information about the size of the tumor and fail to indicate whether the tumor tissue is alive or not. As necrotic tumor tissue requires time to shrink, imaging results always represent delayed information. PET-CT, a combination of CT and positron emission tomography, offers information about the physiological activity of the tumor tissue. Unfortunately, due to the required radiopharmaceuticals with brief half-lives this method is very expensive and, therefore, not suitable for routine monitoring of cancer therapy. Other methods of monitoring the course of a cancer disease or for determining tumor response to treatment are highly dependent on the subjective evaluation of the oncologist.

VEGF-A is part of a complex network of endogenous stimulators and inhibitors of angiogenesis. (Sund et al., 2005, Gastroenterology, 129: 2076-2091). Frequently, angiogenesis and anti-angiogenesis are balanced. However, pro- or anti-angiogenic states are possible, e.g. during wound healing. Drugs may affect the angiogenic balance. Side effects of anti-VEGF-therapy with bevacizumab include arterial ischemic events, wound-healing delays, hemorrhage and bowel perforation (Willet et al., 2005, J. Clin. Oncology, 8136-8138).

Thus, there is a need for improved methods capable of determining if an individual suffers from a cardiovascular disease or a cancer disease. Likewise, there is a need of determining if an individual suffering from cancer also suffers from a cardiovascular disease. Furthermore, there is a need for improved methods for monitoring the course of a cancer disease and the effects of anti-VEGF-therapy. Consequently, the technical problem underlying the present invention must be seen as the provision of means and methods for complying with the aforementioned needs.

The technical problem is solved by the embodiments characterized in the claims and herein below.

The present invention relates to a method for differentiating if a patient suffers from either a cancer disease or a disease selected from a cardiac disease and a cancer disease accompanied by a cardiac disease based on the comparison of the amounts of PIGF or a variant thereof and, if the amount of a PIGF or a variant thereof does not permit to diagnose if the individual suffers from a cancer disease, or from a disease selected from a cardiac disease and a cardiac disease accompanied by a cancer disease, the comparison of the amount of a cardiac troponin or a variant thereof, both determined in a sample of said patient, to reference amounts.

Preferably, the method of the present invention comprises the steps of a) determining the amount of PIGF or a variant thereof and, if the amount of PlGF or a variant thereof does not permit to diagnose if the individual suffers from a cancer disease, or from a disease selected from a cardiac disease and a cardiac disease accompanied by a cancer disease, determining the amount of a cardiac troponin or a variant thereof, both in a sample of a patient; b) comparing the amounts of PlGF and optionally of the cardiac troponin with reference amounts; and c) differentiating between a cancer disease and a disease selected from a cardiac disease and a cardiac disease accompanied by a cancer disease based on the results obtained in step b).

It is also provided a method for differentiating if a patient suffers from either a cancer disease or a disease selected from a cardiac disease and a cancer disease accompanied by a cardiac disease comprising the steps of
a) determining the amount of PlGF or a variant thereof and, if the amount of PlGF or a variant thereof does not permit to diagnose if the individual suffers from a cancer disease, or from a disease selected from a cardiac disease and a cardiac disease accompanied by a cancer disease, determining the amount of a cardiac troponin or a variant thereof, both in a sample of a patient; and
b) comparing the amounts of PlGF and optionally of the cardiac troponin with reference amounts;
whereby it is differentiated between a cancer disease and a disease selected from a cardiac disease and a cardiac disease accompanied by a cancer in the patient.

Accordingly, the present invention relates to a method for differentiating if a patient suffers from either a cancer disease, or a disease selected from a cardiac disease and a cancer disease accompanied by a cardiac disease, comprising the steps of
a) determining the amount of PlGF or a variant thereof in a sample of a patient and comparing it with reference amounts;
b) if the amount of PIGF does not permit to diagnose if the individual suffers from a cancer disease, or from a disease selected from a cardiac disease and a cardiac disease accompanied by a cancer disease, determining the amount of a cardiac troponin or a variant thereof and comparing it with reference amounts; and
c) differentiating between a cancer disease, on the one hand, and a disease selected from a cardiac disease and a cardiac disease accompanied by a cancer disease, based on the results obtained in steps a) and b).

The present invention also provides a method for differentiating between the occurrence of either a cancer disease, or a disease selected from a cardiac disease and a cancer disease accompanied by a cardiac disease, in a patient of which the measured amount of PIGF in a sample of the patient does not permit to diagnose if the individual suffers from a cancer disease or a disease selected from a cardiac disease and a cancer disease accompanied by a cardiac disease, based on the comparison of the amount of a cardiac troponin or a variant thereof determined in a sample of said patient to reference amounts.

Accordingly, the present invention provides a method for differentiating between the occurrence of either a cancer disease, or a disease selected from a cardiac disease and a cancer disease accompanied by a cardiac disease, in a patient of which the measured amount of PIGF in a sample of the patient does not permit to diagnose if the individual suffers from a cancer disease or a disease selected from a cardiac disease and a cancer disease accompanied by a cardiac disease, comprising the steps of
a) determining the amount of a cardiac troponin or a variant thereof and comparing it with reference amounts; and
b) differentiating between a cancer disease and a cancer disease accompanied by a cardiac disease based on the results obtained in step a).

The term "measured amount of PIGF" refers to the case that the PIGF amount has been determined prior to measuring the amount of a cardiac troponin, in order to make the differentiation according to the invention.

The method of the present invention is, preferably, an in vitro method. Moreover, it may comprise steps in addition to those explicitly mentioned above including sample pretreatments or evaluation of the results obtained by the method. The above-described method as well as any other method of the present invention may be carried out manually and/or assisted by automation. Preferably, step (a) and/or (b) may in total or in part be assisted by automation including suitable robotic and sensory equipment for the determination in step (a), and/or a computer-implemented comparison under step (b).

The term "sample" refers to a sample of a body fluid, to a sample of separated cells or to a sample from a tissue or an organ. Samples of body fluids can be obtained by well known techniques and include, preferably, samples of blood, plasma, serum, or urine, more preferably, samples of blood, plasma or serum. Tissue or organ samples may be obtained from any tissue or organ by, e.g., biopsy. Separated cells may be obtained from the body fluids or the tissues or organs by separating techniques such as centrifugation or cell sorting. Preferably, cell-, tissue- or organ samples are obtained from those cells, tissues or organs which express or produce the peptides referred to herein.

Patients with cardiac diseases have elevated levels of PIGF independent of a cancer disease (see example 2) Therefore, the detection of increased amounts of PIGF in samples of patients who suffer from a cardiac disease in addition to cancer have to be interpreted carefully, because the detected PIGF may be the result of the cardiac disease exclusively. The presence of a cardiac disease in a patient is, preferably, diagnosed by comparing the amount of a cardiac troponin or a variant thereof to a reference amount.

As is clear from the foregoing, the present invention comprises determination of the amounts of both PIGF and a cardiac troponin. As the case may be, it may not be necessary to determine the amount of a cardiac troponin after the PIGF amount has been determined. This is in particular the case if the PIGF amount is not elevated in respect to reference amounts, showing that neither cancer nor a cardiac disease should be present in the patient who has been examined.

The term "differentiating between a cancer disease and a disease selected from a cardiac disease and a cancer disease accompanied by a cardiac disease" refers to the process of deciding whether a patient suffers from only a cancer disease or if the patient suffers from either 1) a cardiac disease or 2) a cancer disease accompanied by a cardiac disease. An increased amount of PIGF in a sample of a patient may be caused by a cardiac disease as well as by cancer, with PIGF being non detectable or being within a range below the reference value of PIGF being characteristic for an individual not suffering from a cancer disease and/or a cardiac disease (see below). An amount of PIGF exceeding this range is indicative for cancer disease or a disease selected from a cardiac disease and a cancer disease accompanied by a cardiac disease, as elevated amounts of PIGF can be caused by a cancer disease as well as by a cardiac disease, or even both. Additional diagnostic information is provided by the cardiac troponin. If cardiac troponins are not elevated in respect to the reference amount, this is indicative for the existence of cancer disease without the presence of a cardiac disease. If cardiac troponins are elevated in respect to the reference amount, this is indicative for the presence of a cardiac disease. The cardiac disease may be present in addition to cancer disease, or the cardiac disease may be present without cancer disease existing in the individual.

As will be understood by those skilled in the art, the differentiation according to the method of the present invention is usually not intended to be correct for 100% of the patients to be diagnosed. The term, however, requires that a statistically significant portion of patients can be diagnosed with respect to cancer or cardiac diseases. Whether a portion is statistically significant can be determined without further ado by the person skilled in the art using various well known statistic evaluation tools, e.g., determination of confidence intervals, p-value determination, Student's t-test, Mann-Whitney test, etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001.

The term "patient", preferably, refers to a human. Preferably, the patient does not suffer from systemic bacterial infections or from diseases that cause systemic inflammation other than cancer. Preferably, for the embodiment relating to the differentiation between a cancer disease and a disease selected from a cardiac disease and a cancer disease accompanied by a cardiac disease, the patient is apparently healthy with respect to cardiac diseases, and the patient does not show obvious signs or symptoms of cancer and/or is not diagnosed as having cancer (i.e. he has no known cancer disease and is apparently healthy in respect to cancer disease). However, in a preferred embodiment the patient is suspected to suffer from cancer. For the embodiments relating to the monitoring of the course of a cancer disease or to monitoring anti-cancer therapy or anti-VEGF-therapy, the patient, preferably, shows obvious signs or symptoms of cancer or is diagnosed as having cancer. The patient may be apparently healthy with respect to cardiac diseases, or may not be apparently healthy with respect to cardiac diseases and/or have a known cardiac disease.

In the context of the present invention, the term "apparently healthy" is known to the person skilled in the art and refers to a subject which does not show obvious signs of an underlying disease. In respect to cardiac diseases, this disease here is related to atherosclerotic lesions in the coronary arteries. The subject, thus, may suffer from a cardiac disease as defined below, but preferably does not show obvious signs such that the cardiac disease cannot be diagnosed or assessed without detailed diagnostic examination by a physician. In particular, the diagnosis by a specialist (here: a cardiologist) would be required to diagnose the cardiac disease in the apparently healthy subject not showing obvious symptoms of the disease. In respect to cancer, this disease here is related to any kinds of cancer in the individual. The subject, thus, may suffer from a cancer as defined elsewhere in the specification, but preferably does not show obvious signs such that the cancer disease cannot be diagnosed or assessed without detailed diagnostic examination by a physician. In particular, the diagnosis by a specialist (here: a pathologist) would be required to diagnose the cancer in the apparently healthy subject not showing obvious symptoms of the disease.

The person skilled in the art is aware that the values cited in the context of the present invention ("reference amounts", "reference values", "threshold values") for the cardiac troponins, in particular troponin T, and for PIGF, HGF, sFlT-1 may not apply for patients suffering from impaired renal function, preferably patients suffering from renal failure, in particular patients suffering from chronic and end stage renal failure. In a preferred embodiment of the present invention, patients suffering from impaired renal function, preferably patients suffering from renal failure, in particular patients suffering from chronic and end stage renal failure are not comprised in (excluded from) the methods of the present invention. Preferably, the present invention excludes patients or individuals suffering from impaired renal function, preferably patients suffering from renal failure, in particular patients suffering from chronic and end stage renal failure, more preferably patients with renal hypertension, most preferably all of the patients suffering from one of the diseases and conditions mentioned in this sentence. In this context, "renal failure" is regarded as an impaired glomerular filtration rate (GFR) lying below the usual ranges of 60 to 120 ml/min, preferably below 60 ml/min. Chronic renal failure is a long-standing, progressive deterioration of renal function which often results in end stage renal failure. End stage renal failure is diagnosed when the GFR reaches a rate of up to about 30 ml/min. GFR is determined by the creatinine clearance, which is known to the person skilled in the art. Subjects with impaired renal function show higher levels of troponin I and troponin T than those cited above, due to an impaired clearance of the peptide. The levels vary with the severity of the renal impairment.

The severity of renal impairment is divided into various grades, as displayed below.

| | |
|---|---|
| 0: | ≥ 90 ml/min |
| 1: | ≥ 90 ml/min with microalbuminuria |
| 2: | ≥ 60 - < 90 ml/min |
| 3: | ≥ 30 - < 60 ml/min |
| 4: | ≥ 15 - < 30 ml/min |
| 5: | < 15 ml/min |

(Source: National Kidney Foundation, as published in: Am J. Kidney Dis. 39 suppl. 1, 2002; Clinical Practice Guidelines for chronic kidney disease).

Accordingly, the present invention encompasses a method of differentiating between a cancer disease and a disease selected from a cardiac disease and a cancer disease accompanied by a cardiac disease in a patient, comprising the steps of determining the amount of PIGF or a variant thereof in a sample of a patient and comparing the amount of PIGF with reference amounts; and, if the amount of P1GF is higher than a reference amount indicative for a non-healthy individual suffering from cancer disease and/or a cardiac disease, determining the amount of a cardiac troponin or a variant thereof in a sample of a patient; the differentiation between a cancer disease and a disease selected from a cardiac disease and a cancer disease accompanied by a cardiac disease based on the comparison of the amounts a cardiac troponin with reference amounts.

PIGF amounts may be determined if cancer is suspected in the respective patient. PIGF amounts may also be determined in the course of a routine screening of the patient. In the latter case, the present invention allows to differentiate between a cancer disease and a cardiac disease, by determining the amounts of a cardiac troponin or a variant thereof.

Accordingly, the present invention encompasses a method of differentiating between a cancer disease and a disease selected from a cardiac disease and a cancer disease accompanied by a cardiac disease in a patient who shows PIGF amounts higher than the reference amount for a healthy patient (i.e. indicative for an individual suffering from cancer and/or a cardiac disease), comprising the steps of determining the amount of a cardiac troponin or a variant thereof in a sample of a patient; the differentiation between a cancer disease and a disease selected from a cardiac disease and a cancer disease accompanied by a cardiac disease is based on the comparison of the amounts a cardiac troponin with reference amounts.

The term "cardiac disease", preferably, refers to coronary artery disease (CAD), preferably ischemic coronary artery disease. In the context of the present invention, the individual suffering from CAD may also suffer from heart failure, preferably ischemic heart failure. A "coronary artery disease" as understood here is a disease which is characterized by the partial or complete occlusion of one or more coronary arteries. Preferably, the arteries are blocked by atherosclerotic lesions. Typically, said occlusions result in permanent or transient ischemia of at least a part of the myocardium. Accordingly, the cardiac disease and/or the coronary artery disease is typically accompanied by ischemia. The patient is, preferably, stable, i.e. his/her condition does not rapidly worsen. More preferably, the patient does not suffer from acute coronary syndromes. Even more preferably, the patient is apparently healthy in respect to cardiac diseases.

The presence of a cardiac disease in a patient is, preferably, diagnosed by comparing the amount of a cardiac troponin in a sample of a patient with a reference amount. The amount of a cardiac troponin is, preferably, determined in the first sample according to the method of the present invention. It is to be understood that the method of the present invention also envisages the determination of the cardiac troponin in a separate sample that is taken within a short time before or after the sample for the determination of the amount of PIGF. Preferably said time span does not exceed 1 day, 2 days, 3 days, 4 days, 5, days, 6 days or 7 days.

The term "cancer disease" refers to any type of solid or hematological cancer. More preferably, a "cancer disease" is lung cancer, gastric cancer, colon cancer, breast cancer, ovarian cancer, endometrial cancer, testicular cancer, prostate cancer, skin melanoma, hepatic carcinoma, pancreatic carcinoma, kidney carcinoma, bladder carcinoma, retinoblastoma, neuroblastoma, glioblastoma, osteosarcoma, Ewing's sarcoma, Hodgkin lymphoma, non-Hodgkin lymphoma and leukemia. More preferably, the "cancer disease" is colon carcinoma, non-squamous non-small-cell lung carcinoma, breast carcinoma, kidney cell carcinoma, glioblastoma, pancreas carcinoma or ovarian cancer.

The term "PIGF (Placental Growth Factor)" as used herein refers to a placenta derived growth factor which is a 149-amino-acid-long polypeptide and is highly homologous (53% identity) to the platelet-derived growth factor-like region of human vascular endothelial growth factor (VEGF). Like VEGF, PlGF has angiogenic activity in vitro and in vivo. For example, biochemical and functional characterization of PlGF derived from transfected COS-1 cells revealed that it is a glycosylated dimeric secreted protein able to stimulate endothelial cell growth in vitro (Maqlione, 1993, Oncogene 8(4):925-31). Preferably, PIGF refers to human PIGF, more preferably, to human PIGF having an amino acid sequence as shown in Genebank accession number P49763, GI: 17380553 (Genebank is available from the NCBI, USA under www.ncbi.nlm.nih.gov/entrez).

The term "cardiac Troponin" refers to all Troponin isoforms expressed in cells of the heart and, preferably, the subendocardial cells. These isoforms are well characterized in the art as described, e.g., in Anderson 1995, Circulation Research, vol. 76, no. 4: 681-686 and Ferrieres 1998, Clinical Chemistry, 44: 487-493. Preferably, cardiac Troponin refers to Troponin T and/or Troponin I, and, most preferably, to Troponin T. It is to be understood that isoforms of Troponins may be determined in the method of the present invention together, i.e. simultaneously or sequentially, or individually, i.e. without determining the other isoform at all. Amino acid sequences for human Troponin T and human Troponin I are disclosed in Anderson, loc cit and Ferrieres 1998, Clinical Chemistry, 44: 487-493.

Preferably the biological property of troponin I and its variant is the ability to inhibit actomyosin ATPase, which may e.g. be detected based on the assay described by Moses et al. 1999 PNAS USA 96 (6): 2645-2650). Preferably the biological property of troponin T and its variant is the ability to form a complex with troponin C and I, to bind calcium ions or to bind to tropomyosin, preferably if present as a complex of troponin C, I and T or a complex formed by troponin C, troponin I and a variant of troponin T. PIGF has, preferably, angiogenic activity in vitro and in vivo. For example, biochemical and functional characterization of PIGF derived from transfected COS-1 cells revealed that it is a glycosylated dimeric secreted protein able to stimulate endothelial cell growth in vitro.

The term "variant" encompasses also variants of the specific peptides of the present application. Such variants have at least the same essential biological and immunological properties as the specific cardiac Troponins, HGF, sFlT-1 and PlGF. In particular, they share the same essential biological and immunological properties if they are detectable by the same specific assays referred to in this specification, e.g., by ELISA Assays using polyclonal or monoclonal antibodies specifically recognizing the said cardiac Troponins, HGF, sFlT-1 and PlGF. Moreover, it is to be understood that a variant as referred to in accordance with the present invention shall have an amino acid sequence which differs due to at least one amino acid substitution, deletion and/or addition wherein the amino acid sequence of the variant is still, preferably, at least about 50%, at least about 60%, at least about 70%, at least about 80%, at least about 85%, at least about 90%, at least about 92%, at least about 95%, at least about 97%, at least about 98%, or at least about 99% identical with the amino sequence of the specific Troponin, HGF, sFlT-1 or PlGF, preferably over the entire length of the specific peptide. Variants may be allelic variants or any other species specific homologs, paralogs, or orthologs. Moreover, the variants referred to herein include fragments of the specific cardiac Troponins, HGF, sFlT-1 or PlGF or the aforementioned types of variants as long as these fragments have the essential immunological and biological properties as referred to above. Preferably, the cardiac troponin variants, HGF variants, sFlT-1 variants and PlGF variants have immunological properties (i.e. epitope composition) comparable to those of human troponin T, human troponin I, human HGF, human sFlT-1 or human PlGF. Thus, the variants shall be recognizable by the aforementioned means or ligands used for determination of the amount of the cardiac troponins, HGF, sFlT-1 or PlGF. Such fragments may be, e.g., degradation products of the peptides of the present invention. Further included are variants which differ due to posttranslational modifications such as phosphorylation or myristylation.

In general, for determining the respective amounts/amounts or amount ratios allowing to establish the desired diagnosis in accordance with the respective embodiment of the present invention, ("threshold", "threshold amount", "reference", "reference amount"), the amount/amount(s) or amount ratios of the respective peptide or peptides are determined in appropriate patient groups. The term "reference amount" ("threshold amount") refers, preferably, to an amount of PlGF representing the upper level of normal found in a patient with a cardiac disease but without cancer. The upper limit of normal is, preferably, defined by the 50^{th} percentile, 55^{th} percentile, 60^{th} percentile, 65^{th} percentile, 70^{th} percentile, 75^{th} percentile, 80^{th} percentile, 85^{th} percentile, 90^{th} percentile, or 65^{th} percentile. The term "reference amount" ("threshold amount"), preferably, refers to the amount of a cardiac troponin that allows the differentiation between a patient with and without a cardiac disease. According to the diagnosis to be established, the patient group may, for example, comprise only healthy individuals, or may comprise healthy individuals and individuals suffering from the pathophysiological state which is to be determined, or may comprise only individuals suffering from the pathophysiological state which is to be determined, or may comprise individuals suffering from the various pathophysiological states to be distinguished, by the respective marker(s) using validated analytical methods. In the context of monitoring the course of a disease the groups are formed, preferably, by patients whose disease worsens and by patients whose disease improvers over the course of observation. The results which are obtained are collected and analyzed by statistical methods known to the person skilled in the art. The obtained reference amounts are then established in accordance with the desired probability of suffering from the disease and linked to the particular threshold value. For example, it may be useful to choose the median value, the 60^{th}, 70^{th}, 80^{th}, 90^{th}, 95^{th} or even the 99^{th} percentile of the healthy and/or non-healthy patient collective, in order to establish the threshold value(s), reference value(s) or amount ratios.

Consequently, reference amounts for PIGF can be derived by determining the amount of PIGF in individuals that are healthy with respect to cancer but suffer from a cardiac disease or in patients apparently healthy with respect to both cancer and a cardiac disease. A reference amount for a cardiac troponin is, preferably, derived from a group of patients suffering from a cardiac disease.

A reference amount of a diagnostic marker can be established, and the amount of the marker in a patient sample can simply be compared to the reference value. The sensitivity and specificity of a diagnostic and/or prognostic test depends on more than just the analytical "quality" of the test-they also depend on the definition of what constitutes an abnormal result. In practice, Receiver Operating Characteristic curves, or "ROC" curves, are typically calculated by plotting the value of a variable versus its relative frequency in "normal" and "disease" populations. For any particular marker of the invention, a distribution of marker amounts for subjects with and without a disease will likely overlap. Under such conditions, a test does not absolutely distinguish normal from disease with 100% accuracy, and the area of overlap indicates where the test cannot distinguish normal from disease. A threshold is selected, above which (or below which, depending on how a marker changes with the disease) the test is considered to be abnormal and below which the test is considered to be normal. The area under the ROC curve is a measure of the probability that the perceived measurement will allow correct identification of a condition. ROC curves can be used even when test results don't necessarily give an accurate number. As long as one can rank results, one can create an ROC curve. For example, results of a test on "disease" samples might be ranked according to degree (say 1=low, 2=normal, and 3=high). This ranking can be correlated to results in the "normal" population, and a ROC curve created. These methods are well known in the art. See, e.g., Hanley et al, Radiology 143: 29-36 (1982).

In certain embodiments, markers and/or marker panels are selected to exhibit at least about 70% sensitivity, more preferably at least about 80% sensitivity, even more preferably at least about 85% sensitivity, still more preferably at least about 90% sensitivity, and most preferably at least about 95% sensitivity, combined with at least about 70% specificity, more preferably at least about 80% specificity, even more preferably at least about 85% specificity, still more preferably at least about 90% specificity, and most preferably at least about 95% specificity. In particularly preferred embodiments, both the sensitivity and specificity are at least about 75%, more preferably at least about 80%, even more preferably at least about 85%, still more preferably at least about 90%, and most preferably at least about 95%. The term "about" in this context refers to +/- 5% of a given measurement.

In other embodiments, a positive likelihood ratio, negative likelihood ratio, odds ratio, or hazard ratio is used as a measure of a test's ability to predict risk or diagnose a disease. In the case of a positive likelihood ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group. In the case of a negative likelihood ratio, a value of 1 indicates that a negative result is equally likely among subjects in both the "diseased" and "control" groups; a value greater than 1 indicates that a negative result is more likely in the test group; and a value less than 1 indicates that a negative result is more likely in the control group. In certain preferred embodiments, markers and/or marker panels are preferably selected to exhibit a positive or negative likelihood ratio of at least about 1.5 or more or about 0.67 or less, more preferably at least about 2 or more or about 0.5 or less, still more preferably at least about 5 or more or about 0.2 or less, even more preferably at least about 10 or more or about 0.1 or less, and most preferably at least about 20 or more or about 0.05 or less. The term "about" in this context refers to +/- 5% of a given measurement.

In the case of an odds ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group. In certain preferred embodiments, markers and/or marker panels are preferably selected to exhibit an odds ratio of at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less. The term "about" in this context refers to +/- 5% of a given measurement.

In the case of a hazard ratio, a value of 1 indicates that the relative risk of an endpoint (e.g., death) is equal in both the "diseased" and "control" groups; a value greater than 1 indicates that the risk is greater in the diseased group; and a value less than 1 indicates that the risk is greater in the control group. In certain preferred embodiments, markers and/or marker panels are preferably selected to exhibit a hazard ratio of at least about 1.1 or more or about 0.91 or less, more preferably at least about 1.25 or more or about 0.8 or less, still more preferably at least about 1.5 or more or about 0.67 or less, even more preferably at least about 2 or more or about 0.5 or less, and most preferably at least about 2.5 or more or about 0.4 or less. The term "about" in this context refers to +/- 5% of a given measurement.

The interpretation of the measured amounts of PlGF depends on the presence or absence of cardiac disease in the patient.

Preferably, in a sample of a patient not suffering from a cardiac disease (CAD), the reference amount of PlGF is about 7 pg/ml, about 8 pg/ml, about 9 pg/ml, about 10 pg/ml, about 11 pg/ml or about 12 pg/ml. More preferably, the reference amount of PIGF is the 50^{th} or 75^{th} percentile in patients not suffering from a cardiac disease. Even more preferably, the reference amount is the 75^{th} percentile. Most preferably, the reference amount is about 10.1 pg/ml. The term "about" as used herein refers to +/- 20%, more preferably +/-10%, most preferably, +/- 5% of a given measurement or value. A measured amount of PIGF below said reference amounts is indicative for a healthy subject. A measured amount of PIGF lower than (≤) said reference amount is, preferably, indicative of non existence of a cardiac disease and/or a cancer disease accompanied by a cardiac disease in a patient.

Preferably, the reference amount of PIGF in a sample of a patient suffering from a cardiac disease is about 6 pg/ml, about 7 pg/ml, about 8 pg/ml, about 9 pg/ml, about 10 pg/ml, about 11 pg/ml, about 13 pg/ml or about 15 pg/ml. More preferably, the reference amount is the 25^{th} or 50^{th} percentile determined in patients suffering from a cardiac disease. Even more preferably, the reference amount is the 50^{th} percentile. Most preferably, the reference amount is about 11.3 pg/ml. The term "about" as used herein refers to +/- 20%, more preferably +/-10%, most preferably, +/- 5% of a given measurement or value. If the measured amount of PIGF in a sample of a patient suffering from a cardiac disease is equal to or higher than (≤) the reference amount, the patient suffers from a disease selected from a cardiac disease and a cancer disease accompanied by a cardiac disease. If the measured amount of PIGF in the first sample is below the aforementioned reference amount, this is indicative of a patient not suffering from a cardiac disease.

From the foregoing, it is clear that it is not possible, based on the PIGF amount determined in a individual, to differentiate between an individual suffering from cancer, an individual suffering from a cardiac disease and an individual suffering from cancer accompanied by a cardiac disease.

"Threshold amount" (which may also be referred to as "threshold", "reference" or "reference amount" in the context of the present invention) as used herein with respect to PIGF, preferably, is determined, in one embodiment, in patients with a cardiac disease who are apparently healthy with respect to cancer. In another embodiment, the reference amount is determined in patients apparently healthy with respect to both cancer and cardiac diseases, as defined elsewhere in the specification. A patient is defined as being apparently healthy with respect to said diseases if he does not show symptoms of said diseases. Therefore, the reference amount will in general be derived from subjects known to suffer from a cardiac disease while being apparently healthy with respect to cancer or in subjects who are healthy with respect to cardiac diseases and who do suffer from cancer or in patients apparently health with respect to both cancer and cardiac diseases.

The term "threshold amount" (which may also be referred to as "threshold", "reference" or "reference amount" in the context of the present invention) as used herein with respect to troponin T refers to an amount of troponin T which is measured in patients with cardiac disease who are apparently healthy with respect to cancer. A patient is defined as being apparently healthy with respect to cancer if he does not show symptoms of a cancer disease. Therefore, the reference amount will in general be derived from subjects known to suffer from a cardiac disease while being apparently healthy with respect to cancer or in subjects who are healthy with respect to cardiac diseases and who do suffer from cancer.

A reference amount of troponin T indicative of a cardiac disease in a patient is, preferably about 5.5 pg/ml, about 6.0 pg/ml, about 6.5 pg/ml, about 7.0 pg/ml or about 7.5 pg/ml. More preferably, the reference amount corresponds to the 50^{th} percentile determined in patients suffering from stable coronary artery disease. Most preferably, the reference amount is about 6.6 pg/ml. The term "about" as used herein refers to +/- 20%, more preferably +/-10%, most preferably, +/- 5% of a given measurement or value. An amount of troponin T equal to or higher than (≤) the reference amount is indicative of a cardiac disease in the patient in question.

Determining the amount of PIGF, HGF, sFlT-1 or the amount of a cardiac troponin, preferably troponin T or any other peptide or polypeptide or protein referred to in this specification relates to measuring the amount or concentration, preferably semi-quantitatively or quantitatively. The terms polypeptide and protein are used interchangeable throughout this application. Measuring can be done directly or indirectly. Direct measuring relates to measuring the amount or concentration of the peptide or polypeptide based on a signal which is obtained from the peptide or polypeptide itself and the intensity of which directly correlates with the number of molecules of the peptide present in the sample. Such a signal - sometimes referred to herein as intensity signal -may be obtained, e.g., by measuring an intensity value of a specific physical or chemical property of the peptide or polypeptide. Indirect measuring includes measuring of a signal obtained from a secondary component (i.e. a component not being the peptide or polypeptide itself) or a biological read out system, e.g., measurable cellular responses, ligands, labels, or enzymatic reaction products.

In accordance with the present invention, determining the amount of a peptide or polypeptide can be achieved by all known means for determining the amount of a peptide in a sample. Said means comprise immunoassay devices and methods which may utilize labelled molecules in various sandwich, competition, or other assay formats. Said assays will develop a signal which is indicative for the presence or absence of the peptide or polypeptide. Moreover, the signal strength can, preferably, be correlated directly or indirectly (e.g. reverse- proportional) to the amount of polypeptide present in a sample. Further suitable methods comprise measuring a physical or chemical property specific for the peptide or polypeptide such as its precise molecular mass or NMR spectrum. Said methods comprise, preferably, biosensors, optical devices coupled to immunoassays, biochips, analytical devices such as mass- spectrometers, NMR- analyzers, or chromatography devices. Further, methods include micro-plate ELISA-based methods, fully-automated or robotic immunoassays (available for example on Elecsys^{™} analyzers), CBA (an enzymatic Cobalt Binding Assay, available for example on Roche-Hitachi^{™} analyzers), and latex agglutination assays (available for example on Roche-Hitachi^{™} analyzers).

Preferably, determining the amount of a peptide or polypeptide comprises the steps of (a) contacting a cell capable of eliciting a cellular response the intensity of which is indicative of the amount of the peptide or polypeptide with the said peptide or polypeptide for an adequate period of time, (b) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the amount of the peptide or polypeptide.

Also preferably, determining the amount of a peptide or polypeptide comprises the step of measuring a specific intensity signal obtainable from the peptide or polypeptide in the sample. As described above, such a signal may be the signal intensity observed at an m/z variable specific for the peptide or polypeptide observed in mass spectra or a NMR spectrum specific for the peptide or polypeptide.

Determining the amount of a peptide or polypeptide may, preferably, comprise the steps of (a) contacting the peptide with a specific ligand, (b) (optionally) removing non-bound ligand, (c) measuring the amount of bound ligand. The bound ligand will generate an intensity signal. Binding according to the present invention includes both covalent and non-covalent binding. A ligand according to the present invention can be any compound, e.g., a peptide, polypeptide, nucleic acid, or small molecule, binding to the peptide or polypeptide described herein. Preferred ligands include antibodies, nucleic acids, peptides or polypeptides such as receptors or binding partners for the peptide or polypeptide and fragments thereof comprising the binding domains for the peptides, and aptamers, e.g. nucleic acid or peptide aptamers. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)₂ fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to the peptide or polypeptide. Specific binding according to the present invention means that the ligand or agent should not bind substantially to ("cross-react" with) another peptide, polypeptide or substance present in the sample to be analyzed. Preferably, the specifically bound peptide or polypeptide should be bound with at least 3 times higher, more preferably at least 10 times higher and even more preferably at least 50 times higher affinity than any other relevant peptide or polypeptide. Nonspecific binding may be tolerable, if it can still be distinguished and measured unequivocally, e.g. according to its size on a Western Blot, or by its relatively higher abundance in the sample. Binding of the ligand can be measured by any method known in the art. Preferably, said method is semi-quantitative or quantitative. Suitable methods are described in the following.

First, binding of a ligand may be measured directly, e.g. by NMR or surface plasmon resonance.

Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). Alternatively, the ligand may exhibit enzymatic properties itself and the "ligand/peptide or polypeptide" complex or the ligand which was bound by the peptide or polypeptide, respectively, may be contacted with a suitable substrate allowing detection by the generation of an intensity signal. For measurement of enzymatic reaction products, preferably the amount of substrate is saturating. The substrate may also be labelled with a detectable label prior to the reaction. Preferably, the sample is contacted with the substrate for an adequate period of time. An adequate period of time refers to the time necessary for a detectable, preferably measurable, amount of product to be produced. Instead of measuring the amount of product, the time necessary for appearance of a given (e.g. detectable) amount of product can be measured.

Third, the ligand may be coupled covalently or non-covalently to a label allowing detection and measurement of the ligand. Labelling may be done by direct or indirect methods. Direct labelling involves coupling of the label directly (covalently or non-covalently) to the ligand. Indirect labelling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.). The ligand or substrate may also be "tagged" with one or more tags as known in the art. Such tags may then be targets for higher order ligands. Suitable tags include biotin, digoxigenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a coloured reaction product, fluorescence or chemoluminescence, which can be measured according to methods known in the art (e.g. using a light-sensitive film or a suitable camera system). As for measuring the enzymatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include ³⁵S, ¹²⁵I, ³²P, ³³P and the like. A radioactive label can be detected by any method known and appropriate, e.g. a light-sensitive film or a phosphor imager. Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labelling or other detection methods as described above.

The amount of a peptide or polypeptide may be, also preferably, determined as follows: (a) contacting a solid support comprising a ligand for the peptide or polypeptide as specified above with a sample comprising the peptide or polypeptide and (b) measuring the amount peptide or polypeptide which is bound to the support. The ligand, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand are well known and include, but are not limited to ionic, hydrophobic, covalent interactions and the like. It is also contemplated to use "suspension arrays" as arrays according to the present invention (Nolan 2002, Trends Biotechnol. 20(1):9-12). In such suspension arrays, the carrier, e.g. a microbead or microsphere, is present in suspension. The array consists of different microbeads or microspheres, possibly labelled, carrying different ligands. Methods of producing such arrays, for example based on solid-phase chemistry and photo-labile protective groups, are generally known (US 5,744,305).

Preferably, the amount of PlGF, HGF, sFlT-1 and the amount of a cardiac troponin and, as the case may be, the amounts of other peptides measured in the context of the present invention are determined in a blood sample, e.g., a serum or plasma sample, obtained from a subject as defined in the present invention. Preferably, such a determination is done by ELISA. The amounts of PIGF and troponin T can be determined by the COBAS assay, Roche Diagnostics Mannheim, Germany. The amount of HGF is, preferably, determined with the Quantikine Test, Cat. # DHG00 from R&D Systems, Minneapolis, USA. sFlT-1 is, preferably, determined with the COBAS assay by Roche Diagnostics, Germany.

The term "amount" as used herein encompasses the absolute amount (e.g., of PIGF, HFG, sFlT-1 or a cardiac troponin), the relative amount or concentration (e.g., of PIGF, HFG, sFlT-1 or a cardiac troponin) as well as any value or parameter which correlates thereto. Such values or parameters comprise intensity signal values from all specific physical or chemical properties obtained from the said peptides by direct measurements, e.g., intensity values in mass spectra or NMR spectra. Moreover, encompassed are all values or parameters which are obtained by indirect measurements specified elsewhere in this description, e.g., expression amounts determined from biological read out systems in response to the peptides or intensity signals obtained from specifically bound ligands. It is to be understood that values correlating to the aforementioned amounts or parameters can also be obtained by all standard mathematical operations.

The term "comparing" as used herein encompasses comparing the amount of the peptide, polypeptide, protein comprised by the sample to be analyzed with an amount of a reference source specified elsewhere in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. The comparison referred to in step (b) of the method of the present invention may be carried out manually or computer assisted. For a computer assisted comparison, the value of the determined amount may be compared to values corresponding to references which are stored in a database by a computer program. The computer program may further evaluate the result of the comparison, i.e. automatically provide the desired assessment in a suitable output format. Based on the comparison of the amount(s) determined in step a) to reference amount(s), it is possible to diagnose ischemia, reversible cardiac dysfunction and/or non reversible cardiac injury in said subject. It is to be understood that amounts of PIGF, HGF, sFlT-1 and a cardiac troponin as determined in step (a) of the methods of the presents invention are compared in step (b) to reference amounts for PIGF, HFG. sFlT-1 and a cardiac troponin as specified elsewhere in this application.

Another preferred embodiment of the present invention relates to a method for monitoring anti-VEGF-therapy in a patient based on the comparison of the amount of PIGF or a variant thereof determined in a first sample and in at least one further sample of the patient and, if the amount of PIGF in at least one sample is increased in respect to reference amounts, the amounts of a cardiac troponin or a variant thereof is determined in at least one sample of the individual and compared with a reference amount.

Preferably, the method of the present invention comprises the steps of a) determining the amount of PIGF or a variant thereof in a first sample of the patient; b) determining the amount of PIGF or a variant thereof in at least one further sample of the patient; c) comparing the amount of PIGF or a variant thereof to a reference amount; and, if the amount of PIGF is increased in at least one sample of the patient in respect to reference amounts, determining the amount of a cardiac troponin or a variant thereof, in at least a first sample of the patient and comparing it to reference amounts; and assessing whether or not anti-VEGF-therapy is successful in that patient.

It is also provided a method for monitoring anti-VEGF-therapy in a patient, comprising the steps of
a) determining the amount of PIGF or a variant thereof in a first sample of the patient, and,
b) determining the amount of PIGF or a variant thereof, in at least one further sample of the patient;
c) comparing the amounts of the PlGF or a variant thereof to a reference amount;
d) if the amount of PlGF is increased in at least one sample of the patient in respect to reference amounts, determining the amount of a cardiac troponin or a variant thereof, in at least a first sample of the patient and comparing it to reference amounts;
whereby it is assessed whether or not anti-VEGF therapy is successful based on the results of step c) or steps c) and d).

Accordingly, the present invention relates to a method for monitoring anti-VEGF-therapy in a patient, comprising the steps of
a) determining the amount of PlGF or a variant thereof in a first sample of the patient;
b) determining the amount of PlGF or a variant thereof, in at least one further sample of the patient;
c) comparing the amounts of the PlGF or a variant thereof to a reference amount;
d) if the amount of PlGF is increased in at least one sample of the patient, determining the amount of a cardiac troponin or a variant thereof, in at least a first sample of the patient
e) comparing the determined marker amounts to reference amounts;
f) assessing whether or not anti-VEGF therapy is successful based on the results of step d) or steps d) and e).

The first sample is, preferably, taken immediately before the onset of anti-VEGF-therapy. More preferably, the first sample is taken less than 5 days, less than 4 days, less than 3 days, less than 2 days or less than 1 day before the onset of anti-VEGF-therapy. Also preferably, the first sample is taken shortly after the onset of anti-VEGF-therapy, more preferably less than 1 day, less than 2 days or less than 3 days after the onset of anti-VEGF-therapy.

The at least one further sample is, preferably, taken at least 3 days, at least 4 days, at least 5 days, at least 6 days, at least 7 days, at least 8 days, at least 9 days or at least 10 days after the onset of anti-VEGF-therapy. If more than one further sample is taken, the interval between the further samples is, preferably, at least 7 days, at least 9 days, at least 11 days, at least 13 days or at least 15 days. More preferably, the interval is 7 days.

The term "anti-VEGF-therapy" refers to any therapy that aims at decreasing the amount of VEGF in a patient or at inhibiting the effect of VEGF. Inhibition of the effect of VEGF is achieved, preferably, by blocking the VEGF-receptor with antagonists to VEGF or by binding of a ligand to VEGF. The complex of the ligand and VEGF is unable to bind to the VEGF-receptor. Preferably, anti-VEGF-therapy is used against the formation of new blood vessels (angiogenesis) in a patient suffering from cancer. Once tumors reach a certain size, they require vascularization in order to maintain a sufficient supply with nutrients and oxygen. Therefore, anti-VEGF-therapy is used to starve the tumor. Preferably, anti-VEGF-therapy is directed at VEGF-A or at the VEGF-receptor 1 and/or VEGF-receptor 2. Preferred pharmaceuticals for anti-VEGF-therapy are, preferably, bevacizumab (sold under the trade name "avastin") or ranibizumab (trade name: "lucentis"), sunitinib ("sutent"), vatalanib, sorafinib ("nexavar") and telatinib (also known as BAY 57-9352).

Bevacizumab is a humanized recombinant antibody derived from murine monoclonal antibody VEGF 4.6.1 with approximately 93 % human and 7 % murine protein sequences. Bevacizumab binds to all isoforms of vascular endothelial growth factor A (VEGF-A) while not binding to other family members such as VEGF-B or VEGF-C. Bevacizumab has been shown to neutralize VEGF-A with high affinity (K_{d}=1.1 nM) and inhibits VEGF-induced endothelial cell proliferation in in vitro models. The estimated half-life of bevacizumab is approximately 20 days (range 11 to 50 days). Its clearance depends on tumor burden, weight of the patient and gender (Han, ES and Monk BJ, 2007, Exp. Rev. Anticancer Ther. 7: 1339-1345). By binding to the VEGF-A receptor bevacizumab inhibits the binding of VEGF-A to the receptor. This process is thought to inhibit angiogenesis required for tumor growth (Shord et al., 2009, Am. J. Health Syst. Pharm., 66: 999-1013).

Bevacizumab has been approved by the FDA for the treatment of metastatic colon cancer, non-squamous non-small-cell lung cancer and metastatic breast cancer in combination with chemotherapy. In the European Union avastin is additionally approved for the treatment of advanced kidney cell carcinoma in combination with interferon alpha. In metastatic colon cancer bevacizumab extends progressive free survival from 6.2 to 10.6 months and survival from 15.6 to 20.3 months (Hurwith H. et al., 2004, NEJM, 350: 2335-2342). Similar results were reported for metastatic renal cancer with regard to time to progression but not to survival (Yang JC et al., 2003, NEJM, 349: 427-434).

Anti-VEGF-therapy is, preferably, directed against one or more of the following cancers: colon carcinoma, non-squamous non-small-cell lung carcinoma, breast carcinoma, kidney cell carcinoma, glioblastoma, pancreas carcinoma or ovarian cancer.

The term "VEGF" refers to vascular endothelial growth factor. Four variants of VEGF, VEGF-A, VEGF-B, VEGF-C and VEGF-D, are known to occur naturally in the human body. Preferably, VEGF-A has an amino acid sequence as shown in GenBank accession number NP_001020537. It mediates increased vascular permeability and induces vasculogenesis, angiogenesis and endothelial cell growth. It is frequently found as a disulfide-linked homodimer. VEGF-B has an amino acid sequence as shown in GenBank accession number XP_001128909. While VEGF-A is important for the formation of blood vessels, such as during development or in pathological conditions, VEGF-B seems only to play in role in the maintenance of newly formed blood vessels during pathological conditions (Zhang F et al., 2009, Proc. Natl. Acad. Sci. USA, 106: 6152-6157). VEGF-C has an amino acid sequence as shown in GenBank accession number NP_005420. It is active in angiogenesis and lymphangiogenesis. VEGF-D (also known as c-fos induced growth factor) has an amino acid sequence as shown in GenBank accession number NP_005420. Its function is similar to the function of VEGF-C.

The term "soluble (s)FLT-1" as used herein refers to polypeptide which is a soluble form of the VEGF receptor FLT1. It was identified in conditioned culture medium of human umbilical vein endothelial cells. The endogenous soluble FLT1 (sFLT1) receptor is chromatographically and immunologically similar to recombinant human sFLT1 and binds [125I] VEGF with a comparable high affinity. Human sFLT1 is shown to form a VEGF-stabilized complex with the extracellular domain of KDR/Flk-1 in vitro. Preferably, sFLT1 refers to human sFLT1. More preferably, human sFLT1 can be deduced from the amino acid sequence of Flt-1 as shown in Genebank accession number P17948, GI: 125361. An amino acid sequence for mouse sFLT1 is shown in Genebank accession number BAA24499.1, GI: 2809071. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific sFLT1. Variants may be allelic variants, splice variants or any other species specific homologs, paralogs, or orthologs. Moreover, the variants referred to herein include fragments of the specific sFLT1 or the aforementioned types of variants as long as these fragments have the essential immunological and biological properties as referred to above. Preferably, the sFLT-1 variants have immunological properties (i.e. epitope composition) and/or biological properties comparable to those of human sFLT-1. Thus, the variants shall be recognizable by the aforementioned means or ligands used for determination of the amount of sFLT-1. Such fragments may be, e.g., degradation products of sFLT1. Further included are variants which differ due to posttranslational modifications such as glycosylation, phosphorylation or myristylation. Preferably the biological property of sFLT-1 is the ability to bind to VEGF with a high affinity and/or to form a VEGF-stabilized complex with the extracellular domain of KDR/Flk-1, a process which is found during angiogensis (e.g. following ischemia)..

Hepatocyte growth factor (HGF) was first identified in 1984 and 1985 and purified as a potent mitogen of primary cultured hepatocytes. HGF is single-chain precursor form, and further processing by serine proteases into the two-chain form is coupled to its activation. Serine proteases responsible for the activation of HGF include HGF activator or HGF converting enzyme and urokinase-type plasminogen activator (uPA). The receptor for HGF was identified as a c-met proto-oncogene product. The c-Met receptor is composed of a 50-kDa a-chain and 145-kDa h-chain. Binding of HGF to the c-Met receptor induces activation of tyrosine kinase, resulting in subsequent phosphorylation of C-terminally clustered tyrosine residues. HGF has an organotrophic role in the regeneration and protection of various organs, including the liver, lung, stomach, pancreas, heart, brain, and kidney. Hepatocyte growth factor regulates cell growth, cell motility, and morphogenesis by activating a tyrosine kinase signalling cascade after binding to the proto-oncogenic c-Met receptor. Hepatocyte growth factor is secreted by mesenchymal cells and acts as a multi-functional cytokine on cells of mainly epithelial origin. Its ability to stimulate mitogenesis, cell motility, and matrix invasion gives it a central role in angiogenesis (e.g. following ischemia), tumorogenesis, and tissue regeneration. It is secreted as a single inactive polypeptide and is cleaved by serine proteases into a 69-kDa alpha-chain and 34-kDa beta-chain. A disulfide bond between the alpha and beta chains produces the active, heterodimeric molecule. The protein belongs to the plasminogen subfamily of S1 peptidases but has no detectable protease activity. Alternative splicing of this gene produces multiple transcript variants encoding different isoforms. An amino acid sequence for mouse HGF is shown in Genebank accession number NP034557.2, GI: 46048249. An amino acid sequence for human HGF is shown in Genebank accession number NP000592.3 GI:33859835. Preferably, HGF refers to human HGF. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific HGF. Variants may be allelic variants, splice variants or any other species specific homologs, paralogs, or orthologs. Moreover, the variants referred to herein include fragments of the specific HGF or the aforementioned types of variants as long as these fragments have the essential immunological and biological properties as referred to above. Preferably, the HGF variants have immunological properties (i.e. epitope composition) and/or biological properties comparable to those of human HGF. Thus, the variants shall be recognizable by the aforementioned means or ligands used for determination of the amount of the cardiac troponins. Such fragments may be, e.g., degradation products of HGF. Further included are variants which differ due to posttranslational modifications such as glycosylation, phosphorylation or myristylation. Preferably the biological property of HGF is the ability to bind to the proto-oncogenic c-Met receptor.

The term "monitoring" as used in the context of the present patent application refers to the process of determining whether anti-VEGF-therapy is successful in treating the cancer disease the patient suffers from. Moreover, monitoring according to the method of present invention allows the detection of undesired side-effects, preferably an increased risk of cardiac complications, in the patient receiving anti-VEGF-therapy.

As will be understood by those skilled in the art, the determination of the monitoring of anti-VEGF-therapy according to the method of the present invention is usually not intended to be correct for 100% of the patients to be monitored. The term, however, requires that a statistically significant portion of patients can be diagnosed with respect to successful or unsuccessful anti-VEGF-therapy. Whether a portion is statistically significant can be determined without further ado by the person skilled in the art using various well known statistic evaluation tools, e.g., determination of confidence intervals, p-value determination, Student's t-test, Mann-Whitney test, etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001.

As set forth in the introduction, PlGF stimulates angiogenesis independently of VEGF. Thus, an increase of PlGF after the initiation of anti-VEGF-therapy indicates that the tumor utilizes modes of angiogenesis that are independent of VEGF. Thus, increased amounts of PlGF during anti-VEGF-therapy, indicate "antiangiogenic escape", i.e. formation of angiogenesis factors in response to anti-VEGF therapy. The "antiangiogenic escape" or formation of angiogenesis factors may be a "tumor escape", i.e. a decreasing efficacy or even complete failure of anti-VEGF-therapy. An increase of PlGF which indicates antiangiogenic escape and/or tumor escape is, preferably, statistically significant. Whether an increase is statistically significant can be determined with statistical methods well known in the art and described elsewhere in the patent application. More preferably, an increase of the amount of PlGF to at least about 200 %, at least about 225 %, at least about 250 %, at least about 275 % or at least about 200 % of the amount determined in the first sample indicates antiangiogenic escape and/or tumor escape. Even more preferably the increase indicating antiangiogenic escape and /or tumor escape is at least 250 % or at least about 300 %.

In an embodiment of the present invention, the amount of sFlT-1 is determined in addition to the amount of PlIGF. Whereas PlGF is a marker for angiogenesis (vessel formation), sFlT-1 is a marker for ischemia. It is known to the person skilled in the art that in general ischemia induces angiogenesis. Accordingly, it can be expected that, as the amounts of PIGF increase, the amounts of sFlT-1 increase likewise. Accordingly, it can be expected that both markers give complementary information on the success of the anti-VEGF therapy. Angiogenesis can be induced by a tumor which is treated by anti-VEGF therapy, but can also be induced by a myocardium which becomes ischemic as a consequence of anti-VEGF therapy. Ischemia of the heart is a consequence of sFlT-1 and can be diagnosed by sFlT-1.

It is also possible, in a further embodiment of the present invention, to determine the amount of sFlT-1 in a sample of the patient instead of determining the amount of PlGF.

In an embodiment of the present invention, the amount of HGF is determined in addition to the amount of PlGF and/or sFlT-1. As both markers are independent markers for ischemia, the induction of ischemia in a tissue of the patient is highly probable if the amounts of both markers in the at least on further sample are increased.

It is also possible, in a further embodiment of the present invention, to determine the amount of HGF in a sample of the patient instead of determining the amount of PIGF and/or sFLT-1.

An increase of the amounts of HGF and/or sFlT-1 after the initiation of anti-VEGF-therapy indicates ischemia in the patient. Ischemia is, in principle, a desired effect because anti-VEGF-therapy aims at reducing the blood supply of the tumor, thus starving it. The increases of the amounts of HGF and/or sFlT-1 are, preferably, statistically significant. Whether an increase is statistically significant can be determined with statistical methods well known in the art and described elsewhere in the patent application. More preferably, an increase of the amount of sFlT-1 to at least about 125 %, at least about 130 %, at least about 135 %, at least about 140 %, at least about 150 % or at least about 160 % of the amount determined in the first sample indicates ischemia. Even more preferably the increase indicating ischemia is to at least about 130 % or at least about 150 %. For HGF, an increase to at least about 120 %, at least about 150 %, at least about 200 %, at least about 250 % or at least about 300 % of the amount determined in the first sample is indicative of ischemia. More preferably, the increase of HGF is at least about 120 %.

In a preferred embodiment, the method of the present invention is not limited to determining the efficacy of anti-VEGF-therapy. It, moreover, allows the detection of undesired side effects of said therapy by determining the amount of a cardiac troponin. The side effects of anti-cancer-therapy are, preferably, cardiac complications. Typically, in patients who suffer from a cardiac disease in addition to cancer the risk of such side effects is especially high. The presence of a cardiac disease is, preferably, diagnosed based on the determination of the amount of a cardiac troponin in the first sample of the patient and its comparison with a reference amount. According to the present invention, the determination of the amount of a cardiac troponin is only carried out if the amount of PIGF is increased and/or the amount of sFLT-1 is increased and/or the amount of HGF is increased

Cardiac complications of anti-VEGF-therapy are, preferably, arterial ischemic events. Said events are, preferably, characterized by a newly occurring occlusion of an artery in the heart or by progression of an already existing partial occlusion. As a consequence, parts of the myocardium become ischemic and in cases of prolonged ischemia the ischemic parts of the myocardium die by necrosis. Moreover, permanent ischemia often leads to decreasing performance of the myocardium, i.e. heart failure.

As set forth above, VEGF mediates the formation of collaterals in patients suffering from cardiac disease. In these patients anti-VEGF-therapy potentially leads to undesired side effects because the necessary formation of collaterals in the myocardium is inhibited. If the effect of VEGF is decreased due to anti-VEGF-therapy, an increased expression of PIGF may replace the missing VEGF. Therefore, in patients with cardiac disease, an increase of the amounts of PIGF in the at least one further sample indicates that the myocardium switches from VEGF-mediated angiogenesis to PIGF-mediated angiogenesis. Hence, increasing amounts of PIGF in such a patient, preferably, do not indicate tumor escape but rather antiangiogenic escape. However, if the amount of PIGF in the at least one further sample of a patient suffering from a cardiac disease fails to increase, this is, preferably, indicative of the suppression of the formation of collaterals in the myocardium. Therefore, in patients with cardiac diseases a lacking increase - or even a decrease - of the amount of PIGF is indicative of an increased risk of the patient in question to suffer from cardiac complications of anti-VEGF-therapy.

Moreover, an increase of the amount of the cardiac troponin in the at least one further sample as compared to the first sample indicates myocardial necrosis induced by the anti-VEGF therapy. Whether an increase is statistically significant can be determined with statistical methods well known in the art and described elsewhere in the patent application. More preferably, the amount of the cardiac troponin or the variant thereof increases to at least about 110 %, at least about 120 % or at least about 130 % of the amount in the first sample.

Thus, the cardiac troponin serves two related purposes. The comparison of the amount of the cardiac troponin determined in the first sample with a fix reference amount derived from patients suffering from coronary artery disease or with the upper limit of normal of patients not suffering from coronary artery disease indicates whether the patient suffers from coronary artery disease in addition to cancer. This diagnosis influences the interpretation of PIGF increases or decreases as set forth above. In addition to this, an increase of the cardiac troponin in the at least one further samples indicates increasing myocardial necrosis and, thus, a cardiac side-effect of anti-VEGF-therapy,

Successful anti-VEGF-therapy leads, preferably, to an extended time until tumor progression, to an increased duration of survival or to a cure of the cancer. Whether anti-VEGF-therapy has the aforementioned desired consequences can only be determined after completion of the therapy or after a longer duration of the therapy. Therefore, in the context of the present invention and in a preferred embodiment HGF and sFlt-1 that both indicate ischemia, are applied as surrogate markers for the success of anti-VEGF-therapy. Since the method of the present invention does not allow the direct determination of the response of the tumor to anti-VEGF-therapy, the person skilled in the art knows that monitoring anti-VEGF-therapy according to the method of the present invention, preferably, leads to the identification of those patients, who require closer monitoring with other diagnostic methods with respect to their response to anti-VEGF-therapy. As will be understood by those skilled in the art, such identification is usually not intended to be correct for 100% of the patients to be monitored. The term, however, requires that a statistically significant portion of patients can be diagnosed with respect to a positive or negative response to anti-VEGF-therapy.

Preferably, a successful anti-VEGF-therapy indicates that the current treatment regimen does not need to be adjusted. Conversely, a negative or deteriorating response to anti-VEGF-therapy as indicated by the method of the present invention, preferably, indicates that a closer monitoring of the anti-VEGF-therapy and/or a change of the treatment is required. Preferably, a closer monitoring of anti-VEGF-therapy is performed with PET-CT (computed tomography coupled with positron emission tomography). This method allows for the determination of the metabolic activity of the tumor. Due to its high costs this method is not suitable for routine monitoring of cancer therapy and should be restricted to those patients who especially require a closer monitoring. Such patients are preferably those, who appear to respond poorly to anti-cancer-therapy, preferably anti-VEGF-therapy.

If the method of the present invention indicates an increased risk of cardiac complications as a consequence of anti-VEGF-therapy, anti-VEGF-therapy has to be discontinued or the cardiac health of the patient has to be monitored closely. Close monitoring of the cardiac health of the patient includes, preferably, regular determination of the amounts of NT-proBNP and/or troponin T and/or sonography.

Advantageously, the present invention allows for the early and simple determination of a positive or negative response of a patient to anti-VEGF-therapy. By identifying patients where anti-VEGF-therapy does not induce ischemia or where increases of the amount of PIGF indicate tumor escape it is possible to identify that group of patients which will benefit most from a closer monitoring of anti-VEGF-therapy. Thus, the resources of the health system can be spent on those patients in greatest need. Those patients who apparently respond to anti-VEGF-therapy can spared the hassles of further unnecessary examinations. Moreover, in patients with pre-existing cardiac diseases in addition to cancer an increased risk of cardiovascular complications can be detected. Thus, anti-VEGF-therapy in these patients can be adapted or even discontinued before it leads to cardiac complications.

The present invention also relates to a method for monitoring the course of a cancer disease in a patient based on the comparison of the amounts of PIGF or a variant thereof determined in a first and at least one further sample of the patient, wherein,
i. an increase of the amount of PlGF or the variant thereof in a patient who is apparently healthy with respect to cardiac diseases indicates a progression of the cancer disease;
ii. a decrease of the amount of PlGF or the variant thereof in a patient who is apparently healthy with respect to cardiac diseases indicates a remission of the cancer disease; and
iii. an increase of the amount of PlGF or the variant thereof in a patient suffering from a cardiac disease indicates a progression of the cancer if the amount of PIGF is larger than a reference amount.

The method of the present invention, preferably, comprises the steps of
a) determining the amount of PlGF or a variant thereof in a first sample of the patient;
b) determining the amount of PlGF or a variant thereof in at least one further sample of the patient; and
c) comparing the amounts of PlGF in the at least two samples, wherein
   i. an increase of the amount of PlGF or the variant thereof in a patient who is apparently healthy with respect to cardiac diseases indicates a progression of the cancer disease;
   ii. a decrease of the amount of PlGF or the variant thereof in a patient who is apparently healthy with respect to cardiac diseases indicates a remission of the cancer disease; and
   iii. an increase of the amount of PlGF or the variant thereof in a patient suffering from a cardiac disease indicates a progression of the cancer if the amount of PlGF is larger than a reference amount.

The at least one further sample is, preferably, taken at least 3 days, at least 4 days, at least 5 days, at least 6 days, at least 7 days, at least 8 days, at least 9 days or at least 10 days after the first sample. If more than one further sample is taken, the interval between the further samples is, preferably, at least 7 days, at least 9 days, at least 11 days, at least 13 days or at least 15 days. More preferably, the interval is 7 days.

In the context of the present invention the term "monitoring the course of a cancer disease" refers to the process of determining whether a cancer disease in a patient worsens or improves. In the context of the present invention a progression of the cancer disease is, preferably, indicated by an increase of the amount of PIGF in the at least one further sample of the patient as compared to the first sample, if the patient is healthy with respect to cardiac diseases. It is to be understood that in the context of a continuous monitoring of the course of a cancer disease with more than one further sample the amount of PIGF in each further sample can be compared with any sample taken before the sample in question. Hence, it is possible to detect whether the amount of PIGF in the patient displays a tendency to increase (indicating a progression of the cancer disease in the patient) or whether the amount of PlGF shows a tendency to decrease (indicating a remission of the cancer disease in the patient.

The term "remission of the cancer disease" refers to a temporary or continuous decrease of the symptoms of the cancer disease. Preferably, a remission of the cancer disease is characterized by a decrease of the tumor mass in the patient, the decrease of the rate of tumor growth and/or a redifferentiation of tumor cells. More preferably, one or more tumors in the patient are eradicated completely and permanently.

The term "progression of a cancer disease", preferably, refers to the development of a cancer disease towards a more advanced stage. Disease progression of cancer is typically characterized by at least one of the features selected from the group consisting of formation of metastases (i.e. further systemic spread of the cancer), increase of tumor mass, dedifferentiation of tumor cells, increased growth rate of the tumor cells and local invasion.

The increase or decrease of the amount of PlGF in the at least one further sample is, preferably, statistically significant. Whether an increase or decrease is statistically significant can be determined without further ado by the person skilled in the art using various well known statistic evaluation tools as described above. More preferably, the amount of PlGF increases or decreases to at least about 110 % at least about 120 %, at least about 130 %, at least about 140 %, at least about 150 %, at least about 160 % or at least about 70 % of the amount determined in the first sample. Most preferably, the amount of PIGF increases or decreases by about 130 %.

The term "about" as used herein refers to +/- 20%, more preferably +/-10%, most preferably, +/- 5% of a given measurement or value.

Moreover, the present invention relates to a device for monitoring anti-VEGF-therapy in a patient comprising
a) an analyzing unit for determining the amounts a cardiac troponin or a variant thereof, of PlGF or a variant thereof, and optionally, of HGF or a variant thereof and optionally of sFlT-1 or a variant thereof in a first sample and at least one second sample of the patient and for determining the amount of a cardiac troponin or a variant thereof in a sample of the patient;
b) an evaluation unit for comparing the amounts of a cardiac troponin or a variant thereof, of PlGF or the variant thereof, and optionally of HGF or the variant thereof and optionally of sFlT-1 or the variant thereof in the at least two samples and for comparing the amount of the cardiac troponin to a reference amount.

The term "device" as used herein relates to a system of means comprising at least the aforementioned means operatively linked to each other as to practise the method of the present invention. Preferred means for determining the amounts of the markers of the present invention, and means for carrying out the comparison are disclosed above in connection with the method of the invention. How to link the means in an operating manner will depend on the type of means included into the device. For example, where an analysis unit for automatically determining the amount of the gene products of the present invention is applied, the data obtained by said automatically operating analysis unit can be processed by, e.g., a computer as evaluation unit in order to obtain the desired results. Preferably, the means are comprised by a single device in such a case.

Said device, preferably, includes an analyzing unit for the measurement of the amounts of the peptides of the present invention in an applied sample and an evaluation unit for processing the resulting data. Preferably, the evaluation unit comprises a database with the stored reference amounts and a computer program code which when tangibly embedded on a computer carries out the comparison of the determined amounts and the reference amounts stored in the database. For the comparison of the amounts of the markers of the present invention determined in a first and at least second further sample it is preferred that the evaluation unit comprises storage space for storing the determined amounts. More preferably, the evaluation unit comprises a further computer program code which allocates the result of the comparison to a risk prediction or diagnosis.

Alternatively, where means such as test stripes are used for determining the amounts of the peptides of the present invention, the evaluation unit may comprise control stripes or tables allocating the determined amount to a reference amount. The test stripes are, preferably, coupled to a ligand which specifically binds to the peptides of the present invention. The strip or device, preferably, comprises means for detection of the binding of the peptides of the present invention to the said ligand. Preferred means for detection are disclosed in connection with embodiments relating to the method of the invention above. In such a case, the analysis unit and the evaluation unit are operatively linked in that the user of the system brings together the result of the determination of the amount and the diagnostic or prognostic value thereof due to the instructions and interpretations given in a manual. The analysis unit and the evaluation unit may appear as separate devices in such an embodiment and are, preferably, packaged together as a kit. The person skilled in the art will realize how to link the means without further ado. Preferred devices are those which can be applied without the particular knowledge of a specialized clinician, e.g., test stripes or electronic devices which merely require loading with a sample. The results may be given as output of raw data which need interpretation by the clinician. Preferably, the output of the device is, however, processed, i.e. evaluated, raw data the interpretation of which does not require a clinician. Further preferred devices comprise the analyzing units/devices (e.g., biosensors, arrays, solid supports coupled to ligands specifically recognizing the gene product, Plasmon surface resonance devices, NMR spectrometers, mass-spectrometers etc.) or evaluation units/devices referred to above in accordance with the method of the invention.

Moreover, the present invention relates to a kit for monitoring anti-VEGF-therapy in a patient comprising
a) an analyzing agent for for determining the amount of a cardiac troponin or a variant thereof, of PlGF or a variant thereof, and, optionally of HGF or a variant thereof and optionally of sFlT-1 or a variant thereof in a first sample and at least one second sample of the patient and for determining the amount of a cardiac troponin or a variant thereof in a sample of the patient; and
b) an evaluation unit for comparing the amounts of a cardiac troponin or a variant thereof, of PIGF or the variant thereof, and, optionally, of HGF or the variant thereof and optionally of sFlT-1 or the variant thereof determined in the at least two samples and for comparing the determined amount of the cardiac troponin to a reference amount.

The term "kit" as used herein refers to a collection of the aforementioned components of which may or may not be packaged together. The components of the kit may be comprised by separate vials (i.e. as a kit of separate parts) or provided in a single vial. Moreover, it is to be understood that the kit of the present invention is to be used for practising the methods referred to herein above. It is, preferably, envisaged that all components are provided in a ready-to-use manner for practising the methods referred to above. Further, the kit preferably contains instructions for carrying out the said methods. The instructions can be provided by a user's manual in paper- or electronic form. For example, the manual may comprise instructions for interpreting the results obtained when carrying out the aforementioned methods using the kit of the present invention. The kit shall comprise an analyzing agent. This agent is capable of specifically recognizing the peptides of the present invention in a sample of the subject. Moreover, the said agent shall upon binding to the peptides of the present invention, preferably, be capable of generating a detectable signal, the intensity of which correlates to the amount of the peptides of the present invention present in the sample. Dependent on the type of signal which is generated, methods for detection of the signal can be applied which are well known in the art. Analyzing agents which are preferably used for the kit of the present invention include antibodies or aptamers. The analyzing agent may be present on a test stripe as described elsewhere herein. The amounts of of the peptides of the present invention thus detected can then be further evaluated in the evaluation unit. Preferred evaluation units to be used for the kit of the present invention include those referred to elsewhere herein.

The present invention also relates to the use of a kit or device for determining the amount of PlGF or a variant thereof, the amount of HGF or a variant thereof, the amount of sFlT-1 or a variant thereof and a cardiac troponin or a variant thereof in a sample of a subject, comprising means for determining the amount of PIGF or the variant thereof, the amount of HGF or the variant thereof, the amount of sFlT-1 or the variant thereof and the amount of the cardiac troponin or the variant thereof and/or means for comparing the amount of PIGF or the variant thereof, of HGF or the variant thereof, the amount of sFlT-1 or the variant thereof and the cardiac troponin or the variant thereof to at least one reference amount for: differentiating between cancer and a cardiac disease in a patient; monitoring anti-VEGF-therapy in a patient.

The present invention also relates to the use of: an antibody against a cardiac troponin or a variant thereof, an antibody against HGF or a variant thereof, an antibody against sFlT-1 or a variant thereof and an antibody against a PIGF or a variant thereof and/or of means for determining the amounts of PlGF or a variant thereof, HGF or a variant thereof, sFlT-1 or a variant thereof and a cardiac troponin or a variant thereof and/or of means for comparing the amount of the cardiac troponin or the variant thereof, the amount of HGF or the variant thereof, the amount of sFlT-1 or the variant thereof and of PlGF or the variant thereof to at least one reference amount for the manufacture of a diagnostic composition for: differentiating between cancer and a cardiac disease in a patient; monitoring anti-VEGF-therapy in a patient.

The present invention also relates to the use of: an antibody against PlGF or a variant thereof, an antibody against HGF or a variant thereof, an antibody against sFlT-1 or a variant thereof and an antibody against a cardiac troponin or a variant thereof and/or of means for determining the amounts of PlGF or a variant thereof, of HGF or a variant thereof, of SFlT-1 or a variant thereof and a cardiac troponin or a variant thereof, and/or of means for comparing the amounts of PlGF or the variant thereof, of HGF or the variant thereof, of sFlT-1 or the variant thereof and of the cardiac troponin or the variant thereof to at least one reference amount for: differentiating between cancer and a cardiac disease in a patient; monitoring the course of a cancer disease or monitoring anti-cancer-therapy.

Furthermore, the present invention relates to method for diagnosing an acute cardiovascular event in a patient, wherein the amount of a cardiac troponin is determined in a sample from the patient and compared to a reference amount.

Preferably, the above described method comprises the steps of
a) determining the amount of a cardiac troponin or a variant thereof in a sample of the patient; and
b) comparing the determined amount of the cardiac troponin or the variant thereof to a reference amount, whereby the acute cardiovascular event is recognized.

Thanks to the present invention, PIGF levels in serum - which can be elevated both in cancer diseases as well as in cardiovascular diseases in the same person - can be assigned to cancer disease or cardiovascular disease or both diseases. Dissecting the assignment of PIGF to cancer or cardiovascular disease is established by using troponin T as a specific cardiac biomarker associated with coronary artery disease as well as heart failure. Dissecting PIGF into cancer or cardiovascular associated disease allows a focused approach as to additional diagnostic steps for final diagnosis and finally treatment. In another aspect of the present invention it is shown using sFlT1 (or HGF) that anti-VEGF therapy induces ischemia which is a wanted effect and PlGF in response to ischemia. In a further aspect of the present invention using troponin T, PlGF increases can be assigned to tumor ischemia alone or also to cardiac ischemia resulting from impaired collateral formation due to blockage of VEGF action. This again allows monitoring more precisely the benefits and potential risk of anti-VEGF therapy and thereby improves drug selection in cancer treatment.

All references cited in this specification are herewith incorporated by reference with respect to their entire disclosure content and the disclosure content specifically mentioned in this specification.

The figures show:
**Fig. 1****:** Amounts of HGF after 7 days of treatment versus amounts of sFlT-1 after 7 days of treatment of patients with bevacizumab (10 mg/kg bodyweight) and erlotinib (125 mg/d).
**Fig. 2****:** Amounts of HGF after 7 days of treatment versus amounts of PlGF after 7 days of treatment of patients with bevacizumab (10 mg/kg bodyweight) and erlotinib (125 mg/d).
**Fig. 3****:** Troponin T amounts at baseline versus increase of PlGF during days 1 to 7 of anti-VEGF-therapy with bevacizumab in patients suffering from intestinal tumors. The vertical bar represents a Troponin T amount of 6.6 pg/ml.

The following Examples shall merely illustrate the invention. They shall not be construed, whatsoever, to limit the scope of the invention.

### Examples

### Example 1: Analytical methods

Troponin T was determined with the high sensitive Troponin T immunoassay test by Roche Diagnostics, using the ELECSYS 2010 Analyser. sFlT-1 and NT-proBNP were determined with COBAS immune assays by Roche Diagnostics, Germany to be used with COBAS and ELECSYS analyzers. The tests are based on the sandwich principle and employ two antibodies that are specific for cardiac troponin, sFlT-1 or PIGF, respectively. The first antibody is biotinylated and the second antibody is labelled with ruthenium-complex (Ru(bpy)²⁺₃). Both antibodies are added to the sample to produce the sandwich complex. Thereafter streptavidin-coated microparticles are added to the sample. The biotinylated antibody binds to the streptavidin, thus connecting the sandwich complex to the microparticles. The resulting reaction mixture is then aspirated into a measuring cell where the microparticles are magnetically captured onto the surface of an electrode. After the removal of unbound substances a voltage is applied to the electrode. The voltage induces a chemiluminescence of the ruthenium-complex. This chemiluminescence is measured by a photomultiplier. Its intensity depends on the amount of microspheres with bound sandwich-complexes bound to the electrode. Comparison of the result with a calibration curve gives the concentration of troponin T in the sample. The limit of detection for PIGF is 3 pg/ml. For cardiac troponin T it is 1 pg/ml. For sFlT-1 the limit of detection is 10 pg/ml.

VEGF and HGF were determined with the Quantikine human VEGF assay and the Quantikine human HGF assay by R&D systems, Minneapolis, USA. These tests are based on the sandwich-principle as well. However, compared to the abovementioned tests by Roche Diagnostics the principle is modified. The sample is added to a microtiter well which is coated with an antibody to the respective biomarker. The marker in the sample is, thus, immobilized at the wall of the vessel. Unbound substances are then removed by washing. After that, a second antibody against the marker is added to the well. This antibody is linked to an enzyme. After a further wash to remove any unbound enzyme-conjugated antibody, a substrate solution is added to the well. The enzyme forms a colored reaction product from the substrate. The color development is stopped after a predetermined time and the intensity of the color is measured with a photometer. The concentration of the marker in the sample is calculated by comparing the measured color intensity with a calibration curve. The limit of detection of the VEGF assay is 5.0 pg/ml or 9.0 pg/ml depending on the calibrator diluents used. For the HGF assay the limit of detection is below 40 pg/ml.

### Example 2: PIGF in healthy subjects and in patients with coronary artery disease

PIGF levels were compared in three groups of patients:
A control group consisted of 149 clinically healthy individuals (51 males, mean age 40 years (range 20 to 52 years) and 97 females, mean age 41 years (range 18 to 56 years)). The subjects had normal blood pressure, normal electrocardiogram, no diabetes and no history of cardiac disease or other disorders that would have put them at increased risk of cardiac disorders.

A group of 239 patients with stable coronary artery disease displayed symptomatic but stable heart failure, a normal kidney function (i.e. normal creatinine levels). The group consisted of 186 males and 53 females, mean age 61.2 years. Ischemic heart disease was present in 212 patients, all other patients had non-ischemic heart disease.

| **Table 1:** PIGF amounts [pg/ml] in healthy subjects, subjects with heart failure and subjects with coronary artery disease | | | |
|---|---|---|---|
| Percentile | Healthy Subjects | Coronary artery disease | |
| 75th | 10.13 | 15.88 | |
| 50th | 7.80 | 11.35 | |
| 25th | 6.99 | 6.08 | |

It can be seen that PIGF amounts up to 15.88 pg/ml may be caused by coronary artery disease.

### Example 3: Troponin T in healthy individuals and patients with coronary artery disease

Troponin T was determined in three groups of patients:
The first group consisted of 149 clinically healthy individuals (51 males, mean age 40 years (range 20 to 52 years) and 97 females, mean age 41 years (range 18 to 56 years)). The subjects had normal blood pressure, normal electrocardiogram, no diabetes and no history of cardiac disease or other disorders that would have put them at increased risk of cardiac disorders.

The second group of subjects consisted of individuals obtained from a population based study (i.e. an average, non selected population) in Denmark (Glostrup study, MONICA-10). Samples were taken in 1993 and individuals were 41, 51, 61 und 71 years old at the time of testing. (Olsen et al, 2005, Hypertension, 46: 660-666);Hypertension was present in 26 %, Obesity in 45 % and Hyperinsulinemia in 26 % of the population indicating risk factors for cardiovascular disease. The majority of patients had NT-pro BNP levels within the normal range indicating absence of significant cardiac dysfunction in the majority of individuals (Olsen et al.).

The subjects in the third group suffered from coronary artery disease. A total of 234 patients (122 males, 112 females, mean age 52.1 years) suffering from coronary artery disease were included into the study. All patients were clinically stable for at least 3 weeks prior to the study and they did not show signs and symptoms of chest pain or even acute coronary syndrome. 82 patients had one-vessel disease, 60 had two-vessel disease and 92 had three-vessel disease (determined by angiography) which followed clinical examination, at which also blood was taken by venipuncture. Angiography did not show evidence of acute thrombus formation in any of the patients. All patients had normal kidney function as assessed by creatinine levels within the normal range.

Blood taken by venipuncture was centrifuged within 30 minutes and serum samples were stored at - 20 Celsius until analysed.

| **Table 2:** Cardiac Troponin T [pg/ml] in healthy subjects and in subjects suffering from stable CAD | | | |
|---|---|---|---|
| | Healthy individuals | MONICA-10 | Stable CAD |
| N | 149 | 2652 | 234 |
| 25^{th} Percentile | 0.00 | 0.00 | 1.24 |
| Median | 0.00 | 2.18 | 6.60 |
| 75^{th} Percentile | 0.00 | 5.16 | 23.00 |

Table 2 shows that cardiac troponin is not elevated in healthy individuals and is elevated in virtually all individuals suffering from cardiac diseases. An average population does not show a troponin elevation for all individuals, but for a certain part of them, in accordance with the fact that statistically only a certain portion suffers from cardiac diseases.

### Example 4: VEGF and PlGF in anti-angiogenic therapy of patients with colon cancer

In an observational non-randomized study 13 patients with metastatic colon cancer received treatment with oxaliplatin, fluorouracil and leucovorin (FOLFOX 4) combined with anti-VEGF-therapy bevacizumab 10 mg/kg bodyweight. FOLFOX 4 and bevacuzumab therapy represents a treatment which combines cytotoxic drugs (oxaliplatin, fluorouracil and leucovorin), which are directly cytotoxic to preferably tumor cells with an anti VEGF agent which is inhibiting new vessel growth and thereby contributes using an antiangiogenic mechanism by starvation of tumor cells. (Side effects thus may re related to cytotoxic drungs and/or side effects of antiangiogenesis) .

All patients were clinically stable and asymptomatic with respect to cardiovascular disease. In addition, they had normal kidney function as assessed by creatinine levels within the normal range.

Serum samples were collected immediately before and 2 to 3 weeks after the start of treatment. Samples were kept at -20° C until analysis.

| **Table 3:** VEGF and PIGF in patients with colon cancer receiving FOLFOX 4 and bevacizumab | | | | | | |
|---|---|---|---|---|---|---|
| Sample | Time Point | hsTnT [pg/ml] | PIGF [pg/ml] | PIGF %baseline | VEGF [pg/ml] | VEGF %baseline |
| 1 | 1 | 3.46 | 18 | | 257 | |
| | 2 | | 31 | 179 | 55 | 21 |
| 2 | 1 | 0.0 | 8 | | 226 | |
| | 2 | | 52 | 402 | 166 | 70 |
| 3 | 1 | 2.07 | 24 | | 489 | |
| | 2 | | 58 | 203 | 110 | 26 |
| 4 | 1 | 2.86 | 15 | | 157 | |
| | 2 | | 27 | 174 | 150 | 95 |
| 5 | 1 | 1.71 | 28 | | 60 | |
| | 2 | | 49 | 159 | 97 | 160 |
| 6 | 1 | 12.33 | 22 | | 1074 | |
| | 2 | | 42 | 190 | 114 | 10 |
| 7 | 1 | 8.87 | 18 | | 181 | |
| | 2 | | 28 | 155 | 74 | 40 |
| 8 | 1 | 7.68 | 27 | | 34 | |
| | 2 | | 42 | 161 | 47 | 138 |
| 9 | 1 | 10.31 | 20 | | 314 | |
| | 2 | | 54 | 270 | 59 | 18 |
| 10 | 1 | 33.58 | 26 | | 987 | |
| | 2 | | 90 | 346 | 181 | 18 |
| 11 | 1 | 10.15 | 35 | | 1190 | |
| | 2 | | 43 | 123 | 132 | 11 |
| 12 | 1 | 20.27 | 18 | | 27 | |
| | 2 | | 29 | 161 | 94 | 348 |
| 13 | 1 | 4.11 | 22 | | 380 | |
| | 2 | | 38 | 172 | 70 | 18 |

All 13 patients receiving bevacizumab showed increasing PIGF concentrations 2 to 3 weeks after the initiation of treatment when compared to pre-treatment levels. PIGF levels after 2 to 3 weeks ranged from 123 % to 346 % of the baseline levels. A decrease in VEGF was noted in 9 out of 13 patients, an increase was seen in 3 patients and in a single patient the VEGF level remained virtually unchanged. The decrease of VEGF after anti-VEGF therapy is consistent with the assumption of a response of anti-VEGF, the increase in PIGF after anti-VEGF therapy is considered antiangiogenic escape.

### Example 5: Ischemic response in anti-angiogenic therapy of patients with intestinal tumors

A total of 36 patients with metastatic intestinal tumors received a combination treatment of bevacizumab (10 mg/kg bodyweight) and erlotinib (125 mg/d) in a non-randomized uncontrolled study. In contrast to the combination therapy of cytotoxic agents combined with antiangiogenic agents, treatment of bevacizumab with erlotinib which is an epidermal growth factor inhibitor is solely antiangiogenic. The role of the epidermal growth factor inhibitor is that anti ERGF agents block the production of angiogenic factors such as VEGF but also basic fibroblast growth factor and thus support the efficacy of anti-VEGF agents (Tabernero J, Mol Canc Res 2007: 5: 203 - 220).

All patients were clinically stable and asymptomatic with respect to cardiovascular disease. In addition, they had normal kidney function as assessed by creatinine levels within the normal range. Serum samples were collected immediately before treatment and 7 days after the initiation of treatment and kept at -20 C ° until analysis.

| **Table 4**: VEGF and PIGF in patients with intestinal cancer receiving erlotinib and bevacizumab | | | | | | | |
|---|---|---|---|---|---|---|---|
| | Values at baseline | | | Values after 7 days of treatment | | | |
| | VEGF [pg/ml] | hsTnT [pg/ml] | PIGF [pg/ml] | VEGF [pg/ml] | VEGF %baseline | PIGF [pg/ml] | PIGF %baseline |
| 1308 | 9.00 | 6.22 | 24.24 | 9.00 | 100 | 41.60 | 172 |
| 1311 | 71.47 | 3.08 | 17.26 | 9.00 | 13 | 24.91 | 144 |
| 1312 | 36.69 | 2.96 | 21.06 | 9.00 | 25 | 32.28 | 153 |
| 1315 | 9.00 | 5.68 | 12.94 | 9.00 | 100 | 23.41 | 181 |
| 1318 | 38.07 | 1.25 | 16.30 | 58.38 | 153 | 30.88 | 189 |
| 1319 | 49.78 | 7.93 | 549.23 | 9.00 | 18 | 885.21 | 161 |
| 1322 | 25.40 | 10.16 | 35.11 | 36.93 | 145 | 53.72 | 153 |
| 1325 | 9.00 | 3.00 | 17.86 | 29.38 | 326 | 28.07 | 157 |
| 1329 | 9.00 | 3.00 | 23.56 | 9.00 | 100 | 47.61 | 202 |
| 1332 | 9.00 | 79.33 | 26.90 | 9.00 | 100 | 33.73 | 125 |
| 1334 | 9.00 | 9.35 | 21.73 | 29.68 | 330 | 35.47 | 163 |
| 1335 | 18.09 | 6.78 | 22.97 | 9.00 | 50 | 45.37 | 197 |
| 1336 | 44.93 | 12.31 | 25.34 | 13.57 | 30 | 25.32 | 100 |
| 1344 | 9.00 | 3.41 | 20.05 | 19.87 | 221 | 38.36 | 191 |
| 1349 | 118.19 | 5.20 | 22.41 | 22.64 | 19 | 36.41 | 162 |
| 1350 | 49.78 | 23.40 | 23.69 | 9.00 | 18 | 37.90 | 160 |
| 1351 | 9.00 | 3.35 | 19.36 | 9.00 | 100 | 32.86 | 170 |
| 1355 | 11.42 | 6.50 | 13.37 | 65.00 | 569 | 38.38 | 287 |
| 1359 | 9.00 | 6.76 | 28.59 | 60.75 | 675 | 36.39 | 127 |
| 1362 | 9.00 | 17.03 | 18.27 | 15.09 | 168 | 25.71 | 141 |
| 1395 | 36.46 | 3.09 | 17.68 | 9.00 | 25 | 31.84 | 180 |
| 4202 | 18.30 | 13.39 | 14.20 | 9.00 | 49 | 21.14 | 149 |
| 4203 | 29.34 | 7.47 | 29.11 | 9.00 | 31 | 41.08 | 141 |
| 4204 | 33.97 | 16.08 | 21.83 | 9.00 | 26 | 29.23 | 134 |
| 4205 | 56.60 | 8.88 | 26.36 | 9.51 | 17 | 34.34 | 130 |
| 4206 | 72.91 | 13.23 | 15.67 | 10.45 | 14 | 29.89 | 191 |
| 4207 | 115.82 | 7.23 | 36.44 | 41.59 | 36 | 44.64 | 123 |
| 4210 | 50.89 | 41.96 | 25.07 | 70.29 | 138 | 33.61 | 134 |
| 4214 | 20.25 | 4.11 | 21.15 | 9.00 | 44 | 33.88 | 160 |
| 4216 | 28.32 | 4.19 | 19.39 | 14.35 | 51 | 29.79 | 154 |
| 7004 | 11.46 | 3.77 | 10.42 | 9.00 | 79 | 27.80 | 267 |
| 7005 | 20.25 | 26.44 | 16.20 | 9.00 | 44 | 23.46 | 145 |
| 7006 | 24.17 | 3.25 | 25.87 | 9.00 | 37 | 43.45 | 168 |
| 7013 | 23.19 | 4.67 | 9.20 | 9.00 | 39 | 18.05 | 196 |
| 7023 | 27.11 | 1.64 | 25.75 | 10.49 | 39 | 32.50 | 126 |
| 7024 | 16.34 | 14.56 | 18.08 | 9.00 | 55 | 27.85 | 154 |

Among the 36 patients all but 1 showed an increase of PlGF after 7 days compared to levels at baseline. A single patient did not show changes of PIGF levels. Levels at 7 days varied from 123 % to 287 % of the baseline levels.

VEGF-A decreased in 22 patients and remained unchanged or increased in 14 patients. Amongst these, 4 patients had VEGF levels below the detection limit before treatment was started and VEGF remained below the limit of detection in these patients. After 7 days 20 patients had undetectable levels of VEGF. For calculations VEGF below the limit of detection was calculated with 9.00 pg/ml.

VEGF levels in patients 1318, 1325, 1334, 1344, 1355, 1359, 1362 and 4210 increased during anti-VEGF-therapy. In many of these patients, e.g. patient 1359, VEGF had been below the limit of detection before the onset of therapy. In these patients anti-VEGF-therapy is considered as failed. The decrease of VEGF after anti-VEGF therapy is consistent with the assumption of a response of anti-VEGF, the increase in PIGF after anti-VEGF therapy is considered tumor escape.

In a random subgroup of 31 patients the increases of sFlT-1, HGF and troponin T were determined in addition to the increase of PlGF.

| **Table 5:** Change of sFlT-1, HGF and troponin T in 31 patients receiving bevacizumab and erlotinib | | | |
|---|---|---|---|
| Increase of sFlT-1 [% of baseline level] | Patients with sFlT-1 increase | Patients from column 2 with increase of HGF | Patients from column 2 with increase of troponin T |
| None | 9/31 | 1/9 | 6/9 |
| Up to 130 % | 9/31 | 3/9 | 3/9 |
| 130 % to 150 % | 6/31 | 5/6 | 2/6 |
| Above 150 % | 7/31 | 5/7 | 4/7 |

Some patients showed an increase of troponin T in association with a documented increase in sFlT1 and HGF known to be markers of ischemia, this is explained by the fact that the action of antiVEGF and the VEGF inhibitors is not restricted to the tumor but may affect other parts of the body where (because of pathological mechanisms) angiogenesis is required. Such an organ is the heart and in case of progressed coronary artery disease angiogensis is required for blood supply. As shown troponin T increases were noted as a result of antiangiogenesis therapy which points to the fact that necessary angiogenesis of coronary artery vessels is also blocked which results in an increase of tropoonin T, a specific maker of cardiac necrosis. Troponin T increases have however also been noted without an increase in sFlT1 or HGF. This is because other factors have induced cardiac cell death, such factors include fever, anemia and the like which reduce oxygen supply of the heart because of increased heart rate (fever) or reduced oxygen carrying red blood cells (anemia).

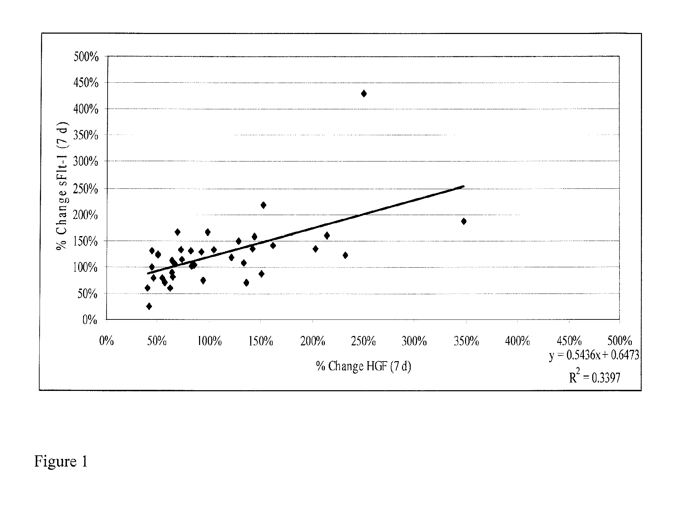

Only in the group of patients having an increase of sFlT-1 to more than 130 % of the baseline level said increase was associated with an increase of HGF in a majority of the patients. Because sFlT-1 and HGF independently indicate ischemia, it is highly probable that anti-VEGF therapy in patients with increased HGF levels and increased sFlT-1 levels was successful in inducing ischemia Figure 1 shows a positive correlation between the increases of HGF and sFlT-1 after 7 days.

Interestingly, the proportion of patients who had increased amounts of troponin T did not show a clear correlation with the induction of ischemia as indicated by HGF and sFlT-1. The highest proportion of patients with increases of troponin T is found amongst those patients without an increase of sFlT-1. Thus, cardiac side effects of anti-VEGF therapy appear to occur independently of the desired anti-tumor effect.

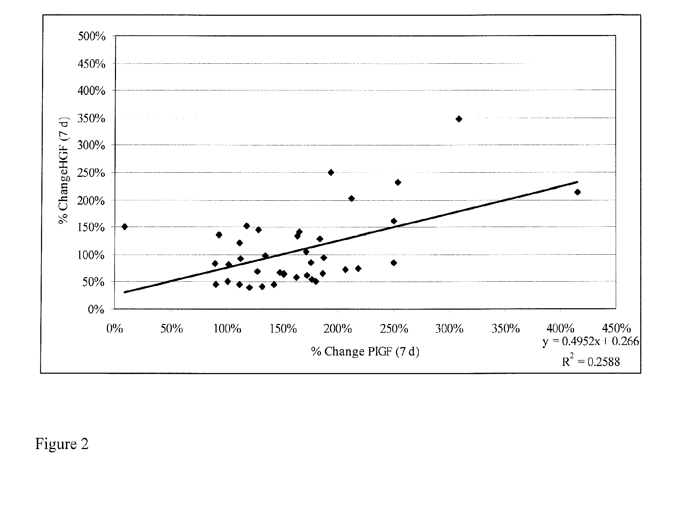

Moreover, a positive correlation between the increases of HGF and of PIGF after 7 days can be observed (Fig. 2). This indicates that the initial ischemia (as indicated by the HGF increase) is followed by increased PIGF mediated angiogenesis.

### Example 6: Correlation of PIGF increase and troponin T at baseline

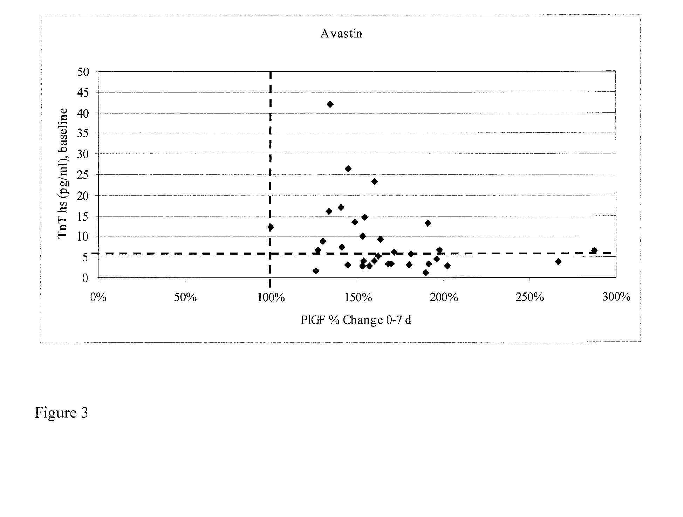

The results of the study of example 5 are also shown in Fig. 3. In Figure 3 Troponin T levels at baseline (which is before treatment with Avastin) is compared to PIGF increase (calculated from the amount of PlGF at baseline (equivalent to 100 %) and the concentration obtained 7 days after the application of the anti-VEGF inhibitor bevacizumab (Avastin)). As can be seen, PIGF increased in all but one patients at 7 days when compared to pre-treatment levels. PIGF increase is considered to be a compensation for reduced angiogenesis due to the application of the VEGF inhibitor Avastin. PIGF increase may be an escape mechanism of the undersupplied tumor ("tumor escape"-). However, PIGF may also increase to compensate the reduction of "wanted angiogenesis" in tissues with reduced perfusion, such as the coronary artery vessels which may require formation of additional vessels to be properly supplied with blood and oxygen (antiangiogenic escape). Troponin T can be used to differentiate between angiogenic escape of the tumor and increased PIGF-mediated angiogenesis in coronary artery vessels, i.e. between a failed cancer therapy and an a proper cancer treatment response accompanied by an adaptive response of the cardiac system which creates new vessels. Troponin T elevations have been found to be associated with coronary artery disease and with resulting heart failure. Significant coronary artery disease and heart failure cannot be associated with Troponin T levels below 6.6. pg/ml (see example 3). Thus, any PIGF increases in patients with Troponin T below 6.6. pg/ml are preferably related to "angiogenic escape", in contrast to cases with Troponin T levels before treatment above 6.6. pg/ml. In the latter cases the presence of cardiovascular disease is likely and a PIGF increase related to a cardiac disease has to be considered in addition to tumor response.

## Claims

1. A method for differentiating if a patient suffers from either a cancer disease or a disease selected from a cardiac disease and a cancer disease accompanied by a cardiac disease comprising the steps of
a) determining the amount of PIGF or a variant thereof and, if the amount of PIGF or a variant thereof does not permit to diagnose if the individual suffers from a cancer disease, or from a disease selected from a cardiac disease and a cardiac disease accompanied by a cancer disease, determining the amount of a cardiac troponin or a variant thereof, both in a sample of a patient; and
b) comparing the amounts of PIGF and optionally of the cardiac troponin with reference amounts;
whereby it is differentiated between a cancer disease and a disease selected from a cardiac disease and a cardiac disease accompanied by a cancer in the patient

2. A method for differentiating between the occurrence of either a cancer disease, or a disease selected from a cardiac disease and a cancer disease accompanied by a cardiac disease, in a patient of which the measured amount of PIGF in a sample of the patient does not permit to diagnose if the individual suffers from a cancer disease or a disease selected from a cardiac disease and a cancer disease accompanied by a cardiac disease, comprising the steps of
a) determining the amount of a cardiac troponin or a variant thereof and comparing it with reference amounts; and
b) differentiating between a cancer disease and a cancer disease accompanied by a cardiac disease based on the results obtained in step a).

3. The method of claim 1, wherein the reference amount for PIGF in a patient without cardiac diseases is 10.1 about pg/ml.

4. A method for monitoring anti-VEGF-therapy in a patient, comprising the steps of
a) determining the amount of PlGF or a variant thereof in a first sample of the patient, and,
b) determining the amount of PlGF or a variant thereof, in at least one further sample of the patient;
c) comparing the amounts of the PlGF or a variant thereof to a reference amount;
d) if the amount of PlGF is increased in at least one sample of the patient, determining the amount of a cardiac troponin or a variant thereof, in at least a first sample of the patient and comparing it to reference amounts;
whereby it is assessed whether or not anti-VEGF therapy is successful based on the results of step c) or steps c) and d).

5. The method according to claim 4, wherein the amount of sFlT-1 is determined in addition to the amount of PlGF.

6. The method according to claim 4 or 5, wherein the amount of HGF is determined in addition to the amount of PlGF and/or sFlT-1.

7. The method of any of claims 5 to 6, wherein increased amounts of HGF and/or sFlT-1 indicate successful anti-VEGF-therapy.

8. The method of any of claims 4 to 7, wherein a stable amount of PlGF or a decreased amount of PlGF indicates undesired side effects of anti-cancer therapy if the amount of the cardiac troponin is higher than a reference amount.

9. The method of any of claims 1 to 8, wherein the cardiac troponin is troponin T.

10. The method of claim 6, wherein the reference amount for troponin T is 6.5 pg/ml.

11. The method of any of claims 1 to 7, wherein the reference amount for PIGF in a patient suffering from a cardiac disease is 11.3 pg/ml.

12. A method for diagnosing an acute cardiovascular event in a patient, comprising the steps of
a) determining the amount of a cardiac troponin or a variant thereof in a sample of the patient; and
b) comparing the determined amount of the cardiac troponin or the variant thereof to a reference amount, whereby the acute cardiovascular event is recognized.

13. Use of antibodies against PlGF or a variant thereof, of antibodies against a cardiac troponin or a variant thereof, and/or of antibodies against HGF or a variant thereof, and/or antibodies against sFlT-1 or a variant thereof for the manufacture of a diagnostic composition for monitoring anti-VEGF-therapy.

14. A device for monitoring anti-VEGF-therapy in a patient comprising
a) an analyzing unit for determining the amounts a cardiac troponin or a variant thereof, of PlGF or a variant thereof, optionally of HGF or a variant thereof and optionally of sFlT-1 or a variant thereof in a first sample and at least one second sample of the patient and for determining the amount of a cardiac troponin or a variant thereof in a sample of the patient; and
b) an evaluation unit for comparing the amounts of a cardiac troponin or a variant thereof, of PlGF or the variant thereof, optionally of HGF or the variant thereof and optionally of sFlT-1 or the variant thereof in the at least two samples and for comparing the amount of the cardiac troponin to a reference amount.

15. A kit for monitoring the course of a cancer disease in a patient comprising
a) an analyzing agent for for determining the amount of a cardiac troponin or a variant thereof, of PlGF or a variant thereof, optionally of HGF or a variant thereof and optionally of sFlT-1 or a variant thereof in a first sample and at least one second sample of the patient and for determining the amount of a cardiac troponin or a variant thereof in a sample of the patient; and
b) an evaluation unit for comparing the amounts of a cardiac troponin or a variant thereof, of PlGF or the variant thereof, optionally of HGF or the variant thereof and optionally of sFlT-1 or the variant thereof determined in the at least two samples and for comparing the determined amount of the cardiac troponin to a reference amount.
